(19) Europäisches Patentamt / European Patent Office / Office européen des brevets

(11) EP 2 272 826 A1

(12) **DEMANDE DE BREVET EUROPEEN**

(43) Date de publication:
**12.01.2011 Bulletin 2011/02**

(21) Numéro de dépôt: **10290344.0**

(22) Date de dépôt: **25.06.2010**

(51) Int Cl.:
*C07C 323/61* (2006.01) *C07C 327/34* (2006.01)
*C07D 205/04* (2006.01) *C07D 211/34* (2006.01)
*C07D 213/55* (2006.01) *C07D 213/73* (2006.01)
*A61P 9/00* (2006.01) *A61K 31/095* (2006.01)
*A61P 7/02* (2006.01)

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **26.06.2009 FR 0903111**

(71) Demandeurs:
- **Les Laboratoires Servier**
  **92284 Suresnes Cedex (FR)**
- **Institut National des Sciences Appliquées de Rouen (INSA)**
  **76801 St-Etienne du Rouvray Cedex (FR)**
- **Centre National de la Recherche Scientifique (C.N.R.S.)**
  **75794 Paris Cedex 16 (FR)**
- **Universite de Rouen**
  **76821 Mont-Saint-Aignan Cédex (FR)**

(72) Inventeurs:
- **Gloanec, Philippe**
  **78160 Marly le Roi (FR)**
- **De Nanteuil, Guillaume**
  **92150 Suresnes (FR)**
- **Parmentier, Jean-Gilles**
  **92130 Issy les Moulineaux (FR)**
- **Guillouzic, Anne-Françoise**
  **92000 Nanterre (FR)**
- **Verbeuren, Tony**
  **78540 Vernouillet (FR)**
- **Rupin, Alain**
  **37510 Savonnieres (FR)**
- **Mennecier, Philippe**
  **78700 Conflans Sainte Honorine (FR)**
- **Vallez, Marie-Odile**
  **93100 Montreuil (FR)**
- **Quirion, Jean-Charles**
  **27310 Bourg-Achard (FR)**
- **Jabault, Philippe**
  **76160 Preaux (FR)**
- **Boyer, Nicolas**
  **74490 Saint Jeoire en Faucigny (FR)**

(74) Mandataire: **Giudicelli, Cathy et al**
**Les Laboratoires Servier**
**Direction Brevets**
**35, rue de Verdun**
**92284 Suresnes Cedex (FR)**

(54) **Dérivés d'acide 2-mercaptocyclopentanecarboxylique, leur procédé de préparation et les compositions pharmaceutiques qui les contiennent**

(57) Composés de formule (I) :

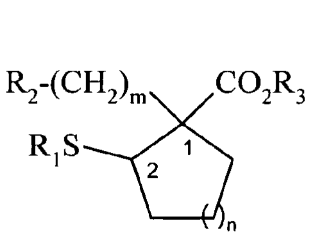

(I),

dans laquelle :
$R_1$ représente un atome d'hydrogène, un groupement de formule $COR_4$,
ou bien $R_1$ représente un groupement de formule (A) :

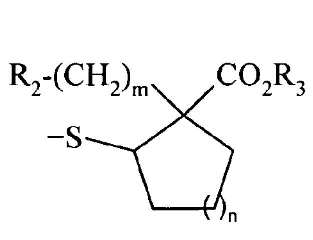

(A),

$R_2$ représente un groupement de formule $NR_5R_6$, ou bien $R_2$ représente un groupement hétérocyclique azoté, aryle ou hétéroaryle,
$R_3$ représente un atome d'hydrogène ou un groupement alkyle,
m représente un entier compris entre 1 et 6,
n représente 0, 1 ou 2,

leurs isomères optiques, ainsi que leurs sels d'addition à un acide pharmaceutiquement acceptable.

EP 2 272 826 A1

## Description

La présente invention a pour objet de nouveaux dérivés d'acide 2-mercapto-cyclopentanecarboxylique, leur procédé de préparation et les compositions pharmaceutiques qui les contiennent.

Les composés de l'invention sont des inhibiteurs du TAFIa (activated thrombin-activatable fibrinolysis inhibitor).

Le TAFI (aussi nommé procarboxypeptidase B plasmatique, procarboxypeptidase R ou procarboxypeptidase U) est une glycoprotéine plasmatique de 60 kDa produite par le foie qui circule sous la forme d'un zymogène. Pendant la coagulation et la fibrinolyse sanguine, la thrombine et la plasmine clivent le prosegment du TAFI au niveau de la liaison Arg92-Ala93 pour le transformer en une enzyme active, le TAFIa, dont la demi-vie est de 8 à 15 minutes à 37°C. Le clivage du prosegment par la thrombine est accéléré par la thrombomoduline, un cofacteur présent dans le plasma et à la surface des cellules vasculaires endothéliales (Bouma BN and Meijers JC, Thrombin-activatable fibrinolysis inhibitor, 2003, Journal of Thrombosis and Haemostasis, 1 : 1566-1574). Le TAFIa régule négativement la fibrinolyse en clivant les résidus lysine C-terminaux des fibres de fibrine qui apparaissent lors de la dégradation partielle de la fibrine par les premières traces de plasmine. Ces résidus lysine C-terminaux sur la fibrine partiellement dégradée se comportent comme des ligands du plasminogène plasmatique circulant et de l'activateur tissulaire du plasminogène (tPA) généré par les cellules endothéliales lors de l'ischémie thrombotique. Ils permettent ainsi de localiser la transformation du plasminogène en plasmine par le tPA sans interférence ni avec l'inhibiteur circulant de la plasmine l'α2-antiplasmine ni avec l'inhibiteur circulant de l'activateur tissulaire du plasminogène (PAI-1). Le clivage des sites lysine C-terminaux par le TAFIa diminue donc la vitesse de génération de la plasmine. La fibrinolyse endogène est alors inhibée et diminue la lyse des thromboses artérielles et veineuses fibrineuses ainsi que la thrombolyse thérapeutique entreprise chez des patients en phase aigue ischémique post-thrombotique. Des inhibiteurs de TAFIa ont donc le potentiel d'augmenter le potentiel fibrinolytique endogène et thérapeutique et de se comporter comme des agents antithrombotiques et profibrinolytiques sans risque hémorragique majeur puisqu'ils n'interfèrent ni avec l'activation plaquettaire ni avec la coagulation lors de l'hémostase sanguine. La propriété d'inhibition du TAFIa permet donc d'envisager l'utilisation des composés de l'invention dans le traitement et la prévention des événements thrombotiques chez les patients à risque.

Leur utilisation sera intéressante dans le traitement, la prévention et la prévention secondaire des complications vasculaires, plus particulièrement cardiovasculaires, pulmonaires et cérébrovasculaires liées aux maladies athérothrombotiques, à l'athérosclérose, au diabète, à l'hyperlipidémie, à l'hypertension, aux maladies veineuses chroniques, au syndrome métabolique lié à l'obésité ou au cancer.

Les composés selon l'invention sont particulièrement utiles pour le traitement, la prévention et la prévention secondaire de l'infarctus du myocarde, de l'angine de poitrine, des accidents vasculaires cérébraux, des anévrismes aortiques, de l'artérite des membres inférieurs, des thromboses veineuses et de l'embolie pulmonaire.

Les facteurs de risques vasculaires et les maladies vasculaires tels que l'hypertension, l'obésité, le diabète, les maladies cardiaques, les maladies cérébrovasculaires et l'hyperlipidémie et donc l'athérosclérose jouent un rôle dans la genèse des démences telles que la maladie d'Alzheimer et la démence vasculaire (Qiu C., De Ronchi D. Et Fratiglioni L., The epidemiology of the dementias : an update, 2007, Current Opinion in Psychiatry, 20 : 380-385). Les composés de l'invention seront donc également utiles pour le traitement et/ou la prévention des démences telles que la maladie d'Alzheimer et la démence vasculaire.

Le TAFIa diminue le potentiel fibrinolytique endogène. En tant qu'inhibiteurs de TAFIa, les composés de la présente invention sont donc utiles pour accompagner le traitement aigu par les fibrinolytiques injectables, tels que le tPA recombinant (par exemple altéplase, tenecteplase, reteplase), l'uPA recombinant ou la streptokinase, qui sont utilisés en situation d'urgence (par exemple infarctus du myocarde, accident vasculaire cérébral).

Les composés de la présente invention renforcent l'activité de ces fibrinolytiques injectables et conduisent donc à leur utilisation avec moins de risques hémorragiques et neurotoxiques (diminution de leur dose et donc réduction de leurs effets secondaires).

La présente invention concerne plus spécialement les composés de formule (I) :

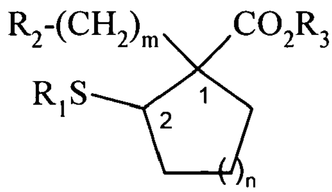

(I),

dans laquelle :

$R_1$ représente un atome d'hydrogène, un groupement de formule $COR_4$ dans laquelle $R_4$ représente un groupement alkyle $C_1$-$C_6$ linéaire ou ramifié ou un groupement aryle,

ou bien $R_1$ représente un groupement de formule (A) :

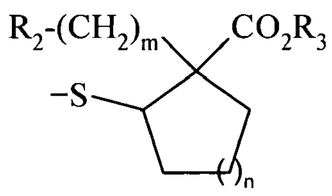

(A),

$R_2$ représente un groupement de formule $NR_5R_6$ dans laquelle $R_5$ et $R_6$, identiques ou différents, représentent chacun un atome d'hydrogène ou un groupement alkyle $C_1$-$C_6$ linéaire ou ramifié, ou bien $R_2$ représente un groupement hétérocyclique azoté, aryle ou hétéroaryle,

$R_3$ représente un atome d'hydrogène ou un groupement alkyle $C_1$-$C_6$ linéaire ou ramifié,

m représente un entier compris entre 1 et 6,

n représente 0, 1 ou 2,

leurs isomères optiques, ainsi que leurs sels d'addition à un acide pharmaceutiquement acceptable.

Par groupement aryle, on entend phényle éventuellement substitué par un ou plusieurs groupements, identiques ou différents, choisis parmi halogène, alkyle ($C_1$-$C_6$) linéaire ou ramifié, alkoxy ($C_1$-$C_6$) linéaire ou ramifié, amino (éventuellement substitué par un ou deux groupements alkyle ($C_1$-$C_6$) linéaire ou ramifié), aminoalkyle, le groupement amino étant éventuellement substitué par un ou deux groupements alkyle ($C_1$-$C_6$) linéaire ou ramifié.

Par groupement hétéroaryle, on entend un groupement aromatique mono- ou bicyclique de 5 à 12 chaînons contenant un, deux ou trois hétéroatomes choisis parmi oxygène, azote ou soufre, étant entendu que l'hétéroaryle peut être éventuellement substitué par un ou plusieurs groupements, identiques ou différents, choisis parmi halogène, alkyle ($C_1$-$C_6$) linéaire ou ramifié, alkoxy ($C_1$-$C_6$) linéaire ou ramifié, amino (éventuellement substitué par un ou deux groupements alkyle ($C_1$-$C_6$) linéaire ou ramifié), aminoalkyle, le groupement amino étant éventuellement substitué par un ou deux groupements alkyle ($C_1$-$C_6$) linéaire ou ramifié.

Parmi les groupements hétéroaryle, on peut citer à titre non limitatif les groupements thiényle, pyridyle, furyle, pyrrolyle, imidazolyle, oxazolyle, isoxazolyle, thiazolyle, isothiazolyle, pyrimidinyle, pyrazinyle, pyridazinyle.

Par groupement hétérocyclique azoté, on entend un groupement monocyclique saturé ou insaturé, de 4 à 7 chaînons contenant un ou plusieurs atomes d'azote, et éventuellement un ou plusieurs autres hétéroatomes choisis parmi l'oxygène et le soufre.

Parmi les groupements hétérocycliques azotés, on peut citer à titre non limitatif les groupements azétidinyle, pyrrolidinyle, pipéridinyle et pipérazinyle.

Par isomères optiques, on entend les diastéréoisomères et les énantiomères.

Les composés de formule (I) possèdent au moins deux centres asymétriques (en positions 1 et 2 du cycle), et peuvent donc exister sous la forme d'un seul énantiomère, d'un seul diastéréoisomère ou sous la forme d'un mélange de diastéréoisomères.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique.

Un aspect de la présente invention concerne les composés de formule (I) pour lesquels $R_1$ représente un atome d'hydrogène.

Un autre aspect de la présente invention concerne les composés de formule (I) pour lesquels $R_2$ représente un groupement amino.

Un autre aspect de la présente invention concerne les composés de formule (I) pour lesquels $R_2$ représente un groupement pyridyle.

Un autre aspect de la présente invention concerne les composés de formule (I) pour lesquels $R_3$ représente

un atome d'hydrogène.

Un autre aspect de la présente invention concerne les composés de formule (I) pour lesquels m représente 3.

Un autre aspect de la présente invention concerne les composés de formule (I) pour lesquels n représente 1.

Un autre aspect de la présente invention concerne les composés de formule (Ia), cas particulier des composés de formule (I) :

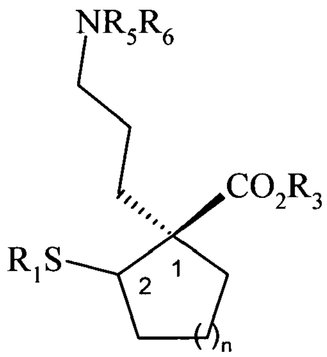

(Ia),

dans laquelle n, $R_1$, $R_3$, $R_5$ et $R_6$ sont tels que définis dans la formule (I).

Un autre aspect de l'invention concerne les composés de formule (I) suivants :

- l'acide (1*R*, 2*S*)-1-(3-aminopropyl)-2-mercapto-cyclopentanecarboxylique, ainsi que ses isomères optiques et ses sels d'addition à un acide pharmaceutiquement acceptable,
- l'acide (1*R*, 2*S*)-2-acétylthio-1-(3-aminopropyl)-cyclopentanecarboxylique, ainsi que ses isomères optiques et ses sels d'addition à un acide pharmaceutiquement acceptable.

La présente invention a également pour objet le procédé de préparation des composés de formule (I), à partir du composé de formule (II) :

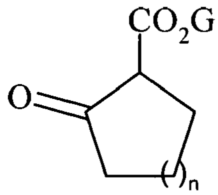

(II),

dans laquelle n représente 0, 1 ou 2, et G représente un groupement protecteur de la fonction carboxy, que l'on met en réaction avec un composé de formule (III) :

$X\text{-}(CH_2)_m\text{-}R'_2$ (III)

dans laquelle X représente un atome d'halogène ou un groupement triflate, tosylate ou mésylate, m représente un entier compris entre 1 et 6,
et $R'_2$ représente un groupement de formule $NR'_5R'_6$ dans laquelle $R'_5$ et $R'_6$, identiques ou différents, représentent chacun un groupement protecteur de la fonction amino ou un groupement alkyle $C_1$-$C_6$ linéaire ou ramifié, ou bien $R'_2$ représente un groupement hétérocyclique azoté éventuellement substitué par un groupement protecteur de la fonction amino ou bien $R'_2$ représente un groupement aryle ou hétéroaryle,
pour conduire au composé de formule (IV) :

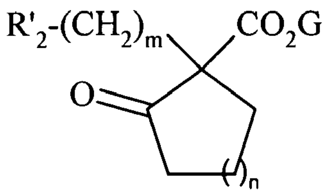

(IV),

dans laquelle R'$_2$, G, m et n sont tels que définis précédemment, que l'on soumet à un agent réducteur de la fonction oxo, pour conduire à un composé de formule (V) :

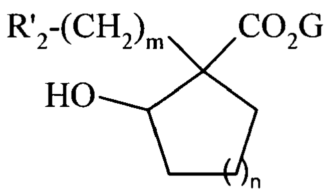

(V),

dans laquelle R'$_2$, G, m et n sont tels que définis précédemment, que l'on met en réaction avec du chlorure de mésyle, du chlorure de tosyle, de l'anhydride triflique ou un réactif d'halogénation, pour conduire au composé de formule (VI) :

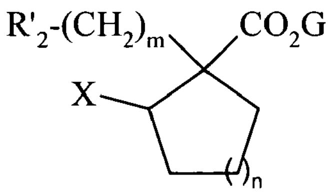

(VI),

dans laquelle R'$_2$, G, m et n sont tels que définis précédemment, et X représente un groupement mésylate, tosylate, triflate ou un atome d'halogène, dont on sépare les diastéréoisomères, lorsque l'on souhaite obtenir un composé de formule (I) sous la forme d'un seul diastéréoisomère, et que l'on fait ensuite réagir avec un composé de formule (VII) :

MS-(CO)-R'$_1$ (VII)

dans laquelle M représente le potassium, le sodium ou le lithium, et R'$_1$ représente un groupement alkyle ou aryle, pour conduire au composé de formule (VIII) :

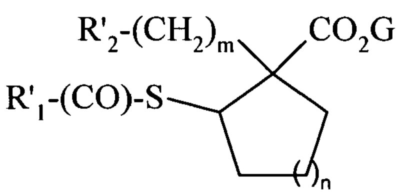

(VIII),

dans laquelle R'$_1$, R'$_2$, G, m et n sont tels que définis précédemment, dont on sépare les énantiomères par chromatographie chirale, lorsque l'on souhaite obtenir un composé de formule (I)

sous la forme d'un seul énantiomère,
et dont on déprotège le cas échéant les fonctions thiol, amino et carboxy, pour conduire au composé de formule (I), que l'on met en réaction, lorsqu'on souhaite obtenir un sel d'addition du composé de formule (I) à un acide pharmaceutiquement acceptable, avec l'acide correspondant.

La présente invention a également pour objet le procédé de préparation des composés de formule (Ia), cas particulier des composés de formule (I) pour lesquels m représente 3 et $R_2$ représente $NR_5R_6$,
à partir du composé de formule (II) :

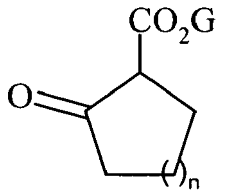

(II),

dans laquelle n représente 0, 1 ou 2, et G représente un groupement protecteur de la fonction carboxy,
que l'on met en réaction avec l'acroléïne,
en présence d'un catalyseur asymétrique tel que, par exemple, le catalyseur Q ou QD, selon que l'on souhaite obtenir le composé de configuration (1R) ou (1S),

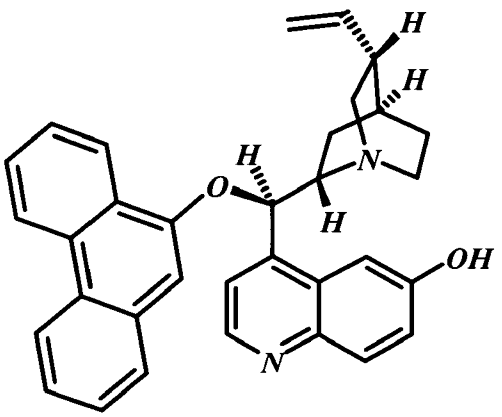

catalyseur Q

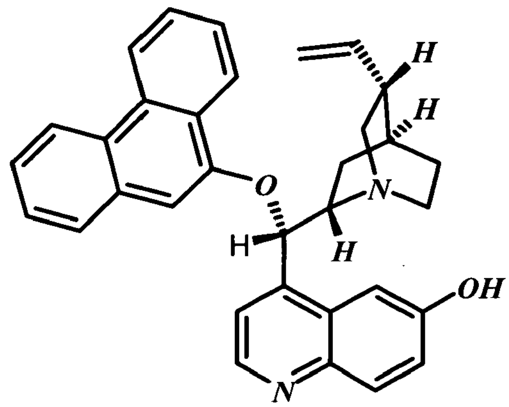

catalyseur QD

pour conduire au composé de formule (IX), de configuration (1R) ou (IS) :

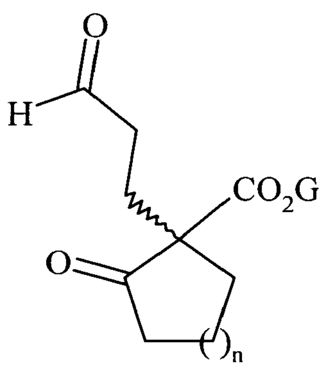

(IX),

dans laquelle n et G sont tels que définis précédemment,
dont on réduit la fonction aldéhyde, pour conduire au composé de formule (X) :

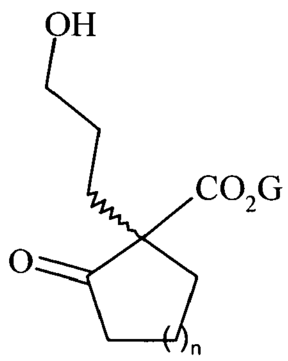

(X),

dans laquelle n et G sont tels que définis précédemment,
que l'on met en réaction avec du chlorure de mésyle, du chlorure de tosyle, de l'anhydride triflique ou un réactif d'halogénation, pour conduire au composé de formule (XI) :

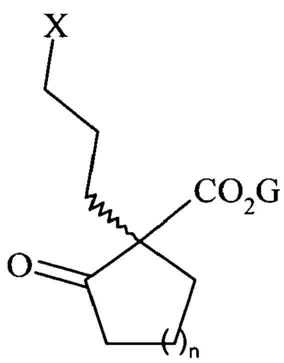

(XI),

dans laquelle n et G sont tels que définis précédemment, et X représente un atome d'halogène ou un groupement triflate, tosylate ou mésylate,

dont on réduit la fonction cétone à l'aide d'un agent réducteur, pour conduire au composé de formule (XII) :

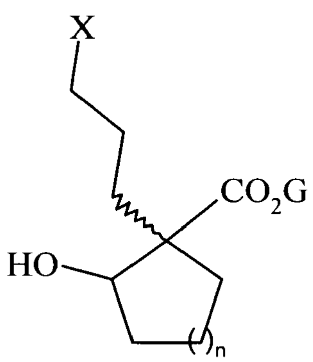

(XII),

dans laquelle n, G et X sont tels que définis précédemment,
que l'on met en réaction avec un composé de formule (XIII) :

$NHR''_5R''_6$ (XIII)

dans laquelle R"$_5$ et R"$_6$ représentent chacun un groupement protecteur de la fonction amino ou un groupement alkyle $C_1$-$C_6$ linéaire ou ramifié,
pour conduire au composé de formule (XIV) :

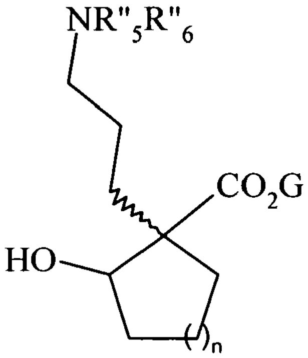

(XIV),

dans laquelle n, G, R"$_5$ et R"$_6$ sont tels que définis précédemment,
que l'on met en réaction avec du chlorure de mésyle, du chlorure de tosyle, de l'anhydride triflique ou un réactif d'halogénation, pour conduire au composé de formule (XV) :

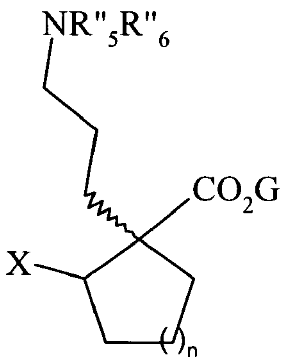

(XV),

dans laquelle n, G, R"$_5$ et R"$_6$ sont tels que définis précédemment, et X représente un groupement mésylate, tosylate, triflate ou un atome d'halogène,
et que l'on fait ensuite réagir avec un composé de formule (VII) :

MS-(CO)-R'$_1$ (VII)

dans laquelle M représente le potassium, le sodium ou le lithium, et R'$_1$ représente un groupement alkyle ou aryle, pour conduire au composé de formule (XVI) :

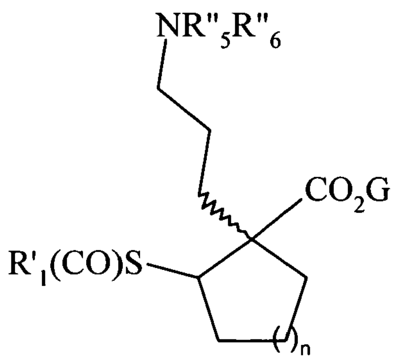

(XVI),

dans laquelle n, R'$_1$, G, R"$_5$ et R"$_6$ sont tels que définis précédemment,
dont on déprotège le cas échéant les fonctions thiol, amino et carboxy, pour conduire aux composés de formule (Ia) :

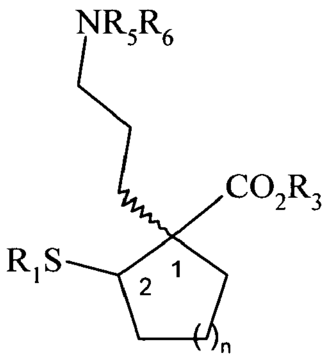

(Ia),

dans laquelle n, $R_1$, $R_3$ $R_5$ et $R_6$ sont tels que définis précédemment,
que l'on met en réaction, lorsqu'on souhaite obtenir un sel d'addition du composé de formule (Ia) à un acide pharmaceutiquement acceptable, avec l'acide correspondant.

Les composés optiquement purs de formule (Ia) sont soit obtenus par réduction asymétrique de la cétone de formule (XI) en utilisant un agent réducteur chiral, soit séparés du mélange de diastéréoisomères à un stade ultérieur.

Les composés de l'invention sont des inhibiteurs de TAFIa.

A ce titre, ils sont utiles dans la prévention ou le traitement des événements thrombotiques chez les patients à risque. Leur utilisation sera intéressante dans le traitement et la prévention des complications vasculaires, plus particulièrement cardiovasculaires, pulmonaires et cérébrovasculaires liées aux maladies athérothrombotiques, à l'athérosclérose, au diabète, à l'hyperlipidémie, à l'hypertension, aux maladies veineuses chroniques, au syndrome métabolique lié à l'obésité ou au cancer.

Les composés selon l'invention sont particulièrement utiles pour le traitement, la prévention et la prévention secondaire de l'infarctus du myocarde, de l'angine de poitrine, des accidents vasculaires cérébraux quelle que soit leur origine (notamment athérothrombotique, cardioembolique ou causée par une fibrillation auriculaire), des anévrismes aortiques ou de l'artérite des membres inférieurs, des thromboses veineuses (notamment chez le patient cancéreux cathétérisé) et de l'embolie pulmonaire.

Les facteurs de risques vasculaires et les maladies vasculaires tels que l'hypertension, l'obésité, le diabète, les maladies cardiaques, les maladies cérébrovasculaires et l'hyperlipidémie et donc l'athérosclérose jouent un rôle dans la genèse des démences telles que la maladie d'Alzheimer et la démence vasculaire (Qiu C., De Ronchi D. Et Fratiglioni L., The epidemiology of the dementias : an update, 2007, Current Opinion in Psychiatry, 20 : 380-385). Les composés de l'invention seront donc également utiles pour le traitement et/ou la prévention des démences telles que la maladie d'Alzheimer et la démence vasculaire.

Le TAFIa diminue le potentiel fibrinolytique endogène. En tant qu'inhibiteurs de TAFIa, les composés de la présente invention sont donc utiles pour accompagner le traitement aigu par les fibrinolytiques injectables, tels que le tPA recombinant (par exemple altéplase, tenecteplase, reteplase), l'uPA recombinant ou la streptokinase, qui sont utilisés en situation d'urgence (par exemple infarctus du myocarde, accident vasculaire cérébral).

Les composés de la présente invention renforcent l'activité de ces fibrinolytiques injectables et conduisent donc à leur utilisation avec moins de risques hémorragiques et neurotoxiques (diminution de leur dose et donc réduction de leurs effets secondaires).

La présente invention a également pour objet les compositions pharmaceutiques contenant un composé de formule (I), en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

Outre le composé de formule (I), les compositions pharmaceutiques selon l'invention contiennent un ou plusieurs excipients ou véhicules tels que des diluants, des lubrifiants, des liants, des agents de désintégration, des absorbants, des colorants, des édulcorants.

A titre d'exemple d'excipients ou véhicules, on peut citer :

- *pour les diluants :* le lactose, le dextrose, le sucrose, le mannitol, le sorbitol, la cellulose, la glycérine,
- *pour les lubrifiants :* la silice, le talc, l'acide stéarique et ses sels de magnésium et de calcium, le polyéthylène glycol,
- *pour les liants :* le silicate d'aluminium et de magnésium, l'amidon, la gélatine, la tragacanthe, la méthylcellulose, la carboxyméthylcellulose de sodium et la polyvinylpyrrolidone,
- *pour les désintégrants* : l'agar, l'acide alginique et son sel de sodium, les mélanges effervescents.

Le pourcentage de principe actif de formule (I) dans la composition pharmaceutique est préférentiellement compris entre 5% et 50% en poids.

La posologie utile varie selon l'âge et le poids du patient, la voie d'administration, la nature et la sévérité de l'affection, et la prise de traitements éventuels associés et s'échelonne de 0,5 mg à 1000 mg en une ou plusieurs prises par jour.

Selon un aspect de la présente invention, les compositions pharmaceutiques selon la présente invention ne contiennent pas d'autre principe actif que le composé de formule (I).

Selon un autre aspect de la présente invention, les compositions pharmaceutiques selon la présente invention, outre le composé de formule (I), contiennent également un fibrinolytique, plus particulièrement un fibrinolytique injectable tel que le tPA recombinant (par exemple altéplase), l'uPA recombinant
ou la streptokinase. Dans ce cas, les compositions sont sous forme injectable.

L'échelle de dose du tPA pourra varier entre 0 et 100 mg.

Selon un autre aspect de la présente invention, les compositions pharmaceutiques selon la présente invention, contiennent, outre le composé de formule (I), un anticoagulant tel que, par exemple, la warfarine, le dabigatran etexilate, le rivaroxaban.

L'échelle de dose de la warfarine pourra varier entre 1 et 100mg.

Selon un autre aspect de la présente invention, les compositions pharmaceutiques selon la présente invention, contiennent, outre le composé de formule (I), un antiplaquettaire tel que, par exemple, l'aspirine, le clopidogrel , le prasugrel....

L'échelle de dose de l'aspirine pourra varier entre 10 et 1000 mg.

L'échelle de dose du clopidogrel pourra varier entre 10 et 1000 mg.

Les exemples suivants illustrent la présente invention. Les structures des composés décrits dans les exemples ont été déterminées selon les techniques spectrophotométriques usuelles (infrarouge, résonance magnétique nucléaire, spectrométrie de masse).

**ABREVIATIONS**

| | |
|---|---|
| (+)-DIPCl : | (+)-diisopinocamphéylchloroborane ((+)-diisopinocamphenyl boron chloride) |
| DMAP : | diméthylaminopyridine |
| DMF : | diméthylformamide |
| DMSO : | diméthylsulfoxyde |
| eq : | équivalent molaire |
| HMPA : | hexaméthylphosphoramide |
| HPLC : | Chromatographie en Phase Liquide à Haute PerformanceRMN : Résonance Magnétique Nucléaire |
| TAFIa : | activated thrombin-activatable fibrinolysis inhibitor |
| THF : | tétrahydrofurane |
| tPA : | activateur tissulaire du plasminogène (**t**issue **p**lasminogen **a**ctivator) |
| uPa : | activateur du plasminogène de type urokinase (**u**rokinase **p**lasminogen **a**ctivator) ou urokinase |

**EXEMPLE 1 : Acide (1R, 2S)- 1-(3-aminopropyl)-2-mercaptocyclopentanecarboxylique, trifluoroacétate**

*Stade A : 2-Oxo-cyclopentanecarboxylate de benzyle*

Dans un ballon de 4L muni d'un appareil de Dean-Stark et d'un réfrigérant, le 2-oxo-cyclopentanecarboxylate de méthyle (380 mL ; 3 mol), l'alcool benzylique (342 mL; 3,3 mol; 1,1 eq ) et la DMAP (18,3g; 0,15 mol; 0,05 eq) sont dissous dans 1800mL de cyclohexane. Le milieu réactionnel est agité 48 heures au reflux (température du milieu : 90°C) et le méthanol formé est distillé. Après refroidissement, le milieu réactionnel est concentré, repris par 1500 mL de dichlorométhane. La phase organique est lavée à l'acide chlorhydrique N, à l'eau, puis par une solution saturée de chlorure de sodium. Elle est ensuite séchée, filtrée puis évaporée. L'huile ainsi obtenue est purifiée par distillation fractionnée sous pression réduite (128 - 130°C / $4.10^{-2}$ mbar) pour conduire au produit attendu.

*Stade B : 1-(4-tert-Butoxy-4-oxobutyl)-2-oxo-cyclopentanecarboxylate de benzyle*

On coule, à température ambiante, sous vive agitation, une solution de 2-oxo-cyclopentanecarboxylate de benzyle obtenu au stade précédent (1g; 5,43 mmol) dans 5 mL d'acétone anhydre, à une suspension de carbonate de potassium sec ( 3g ; 21,7mmol ; 4 eq) dans 10 mL d'acétone anhydre. Le milieu réactionnel est maintenu sous vive agitation pendant une heure, puis on coule rapidement, le 4-bromobutanoate de tert-butyle (2,04 g; 9,16 mmol; 2eq) en solution dans 2 mL d'acétone. Le milieu réactionnel est porté au reflux pendant une nuit. Après refroidissement, le milieu réactionnel est filtré puis repris par 20 mL dichlorométhane. La phase organique est lavée, séchée, filtrée puis évaporée.
Le produit brut est purifié par flash chromatographie sur gel de silice en utilisant comme éluant un mélange heptane /acétate d'éthyle (95/5).

*Stade C : Acide 4-{1-[(benzyloxy)carbonyl]-2-oxocyclopentyl)butanoïque*

A une solution de l'ester de *tert*-butyle obtenu au stade précédent (200g; 0,545 mol) dans 700 mL de dichlorométhane anhydre, on coule, à température ambiante, en vingt minutes, 140 mL d'acide trifluoroacétique. Le milieu réactionnel est maintenu, sous agitation, une nuit à température ambiante.
Rq : Ajouter 10% d'acide trifluoroacétique supplémentaire si après une nuit d'agitation il reste du produit de départ et maintenir l'agitation pendant 3 heures.
Le milieu réactionnel est évaporé, repris au toluène (3x500ml) afin d'éliminer le maximum d'acide trifluoroacétique. Après séchage sous vide de pompe, l'huile brute obtenue est reprise par une solution d'hydrogénocarbonate de sodium (84g / 1L; 2eq). La phase aqueuse basique est alors lavée à l'éther (3x 300mL) puis ré-acidifiée par un solution d'acide chlorhydrique 4N. La phase aqueuse acide est extraite à l'acétate d'éthyle (3x300mL). Les phases organiques regroupées sont lavées, séchées et évaporées. Le produit est utilisé sans purification dans l'étape suivante.

*Stade D : 1-(3-{[Benzyloxy)carbonyl]amino}propyl)-2-oxo-cyclopentanecarboxylate de benzyle*

A une solution d'acide de l'étape précédente (123,7g; 0,406 mol) dans 1L de toluène anhydre, on ajoute, goutte à goutte en 5 minutes, à température ambiante, la triéthylamine (84,5 mL; 0,610 mol; 1,5eq), l'alcool benzylique (75,6mL; 0,731 mol; 1,8eq), puis le diphénylphosphorylazide (96,3 mL; 0,447 mol; 1,1eq). Le milieu réactionnel est agité au reflux pendant une nuit.
Après refroidissement, le milieu réactionnel est évaporé repris par 1L d'acétate d'éthyle. La phase acétate d'éthyle est lavée par une solution d'acide chlorhydrique N (3x200mL), à l'eau, par une solution saturée d'hydrogénocarbonate de sodium puis par une solution saturée de chlorure de sodium. La phase organique est séchée, filtrée puis évaporée.
Le produit brut est purifié en distillant les impuretés (majoritairement de l'alcool benzylique) de 50 à 70°C sous $4.10^{-2}$ mbar.

*Stade E : 1-(3-{[benzyloxy)carbonyl]amino}propyl)-2-hydroxy-cyclopentanecarboxylate de benzyle*

A une solution du céto-ester de l'étape précédente (98g; 0,239 mol) dans 600 mL de méthanol anhydre, on ajoute , à -10°C , en dix fractions le borohydrure de sodium (11,4g ; 0,301 mol; 1,25eq). On maintient le milieu réactionnel à -10°C pendant une heure, puis laisse remonter et agiter à température ambiante pendant deux heures. Après évaporation du méthanol, le milieu réactionnel est repris par 1L l'acétate d'éthyle. La phase organique est lavée par une solution de chlorure d'ammonium à 10%, à l'eau puis par une solution saturée de chlorure de sodium. La phase organique est séchée, filtrée puis évaporée.

*Stade F : 1-(3-{[(benzyloxy)carbonyljamino}propyl)-2-[(méthylsulfonyl)oxy]cyclopentanecarboxylate de benzyle (diastéréoisomère trans racémique)*

A une solution de l'alcool obtenu à l'étape précédente (98g ; 0,238 mol) dans 950 mL de tétrahydrofurane anhydre, on ajoute, à température ambiante, la triéthylamine (49,53 mL ; 0,357 mol; 1,5eq). On refroidit le milieu réactionnel à -30°C, puis coule goutte à goutte, le chlorure de mésyle (27,61 mL ; 0,357 mol ; 1,5eq) en solution dans 120 mL de tétrahydrofurane anhydre. On laisse remonter et agiter à température ambiante pendant deux heures. Après évaporation du tétrahydrofurane, le milieu réactionnel est repris par 1L l'acétate d'éthyle. La phase organique est lavée, séchée, filtrée puis évaporée.

Le produit brut est purifié par flash chromatographie sur gel de silice (5Kg) en utilisant comme éluant un gradient heptane /acétate d'éthyle (7/3 à 6/4).

Le diastéréoisomère trans est le premier dans l'ordre d'élution.

*<u>Stade G</u> : 2-(Acétylthio)-1-(3-{[(benzyloxy)carbonyl]amino}propyl)cyclopentanecarboxylate de benzyle (diastéréoisomère cis racémique)*

*<u>Préparation du thioacétate de potassium :</u> Très hygroscopique - Ne pas sécher - A préparer et utiliser extemporanément.*

On porte à reflux à une suspension de 40g de thioacétate de potassium dans 350 mL d'acétonitrile anhydre pendant 30 minutes , filtre puis renouvelle l'opération une deuxième fois.
A une solution de mésylate (racémique trans) de l'étape précédente (25 g ; 0,051 mol) dans 800ml d'acétonitrile anhydre, on ajoute le thioacétate de potassium préalablement lavé (45g non essoré - théorie :29,2g; 0,255mol; 5eq), de l'éther couronne 18C6 (13,5g; 0,051 mol; 1 eq) puis porte le milieu réactionnel à reflux pendant vingt heures. Après refroidissement, on filtre le milieu réactionnel puis évapore le solvant.
Le produit brut est purifié par flash chromatographie sur gel de silice (2Kg) en utilisant comme éluant un gradient heptane /acétate d'éthyle (8/2 à 5/5).
Rq : cette première colonne permet une pré-purification et la récupération du produit de départ n'ayant pas réagi.
Le lot contenant le produit attendu (6,5g) est purifié par flash chromatographie sur gel de silice (200g) en utilisant comme éluant un gradient dichlorométhane / acétate d'éthyle (99/1 à 95/5).

*<u>Stade H</u> : (1R, 2S)- 2-(Acétylthio)-1-(3-{[(benzyloxy)carbonyl]amino}propyl)cyclopentanecarboxylate de benzyle*

Les énantiomères du composé cis racémique obtenu au stade précédent sont séparés par chromatographie chirale préparative sur une colonne CHIRALPAK AD-H 5 μm en utilisant comme phase mobile un mélange $CO_2$/EtOH (80/20).
La longueur d'onde de détection est de 230 nm.
L'énantiomère *(1R, 2S) = énantio* 2 est le *deuxième* dans l'ordre d'élution.

*<u>Stade I</u> : (1R, 2S)- 1-(3-{[(Benzyloxy)carbonyl]amino}propyl)- 2-mercaptocyclopentane-carboxylate de benzyle*

A une solution du composé obtenu à l'étape précédente (8,16g ; 0,0174 mol) dans150 mL de dioxane anhydre et dégazé, on ajoute, à 10°C , goutte à goutte, une solution de soude N (35 mL; 0,0348 mol; 2eq). On chauffe le milieu à 60°C pendant 30minutes. Après refroidissement, on neutralise le milieu réactionnel par addition de 35 mL d'acide chlorhydrique N, puis lyophilise.

*<u>Stade J</u> : Acide(1R, 2S)- 1-(3-aminopropyl)-2-mercaptocyclopentanecarboxylique, trifluoroacétate*

Dans un réacteur de 2L, on condense 600mL d'ammoniac, on ajoute , à -78°C , en dix fractions le sodium (10g ; 0,435 mol; 25eq). On maintient le milieu réactionnel à -78°C pendant 45 minutes. On additionne alors, goutte à goutte en 30 minutes, une solution du composé obtenu à l'étape précédente dans un mélange tétrahydrofurane/méthanol (140mL/12mL) préalablement dégazé. On maintient l'agitation à -78°C pendant 1 heure puis laisse remonter et agiter à température ambiante. Après élimination de l'ammoniac, on ajoute au milieu réactionnel en 30 minutes une solution de chlorure d'ammonium (23,3g dans 110 mL d'eau), évapore le tétrahydrofurane puis acidifie par une solution d'acide chlorhydrique 6M (15 mL).On filtre puis lyophilise.
Le produit brut est désalé par HPLC préparative sur colonne Kromasil en utilisant comme phase mobile un gradient eau/acétonitrile/acide trifluoroacétique (100/0/0,1 pendant 10 minutes puis 100/0/0,1 à 70/30/0,1 en 20 minutes suivi d'un isocratique 70/30/0,1).
On lyophilise les fractions contenant le produit attendu ainsi que le dimère correspondant (pont disulfure).
A une solution du lyophilisat précédent (4,3g) dans 100 mL d'acide acétique, on ajoute 4,5g de zinc (10eq) puis sous vive agitation, porte le milieu réactionnel à 60°C jusqu'à disparition du dimère (environ 6 heures). Après refroidissement, on filtre le milieu réactionnel, dilue le filtrat avec 400mL d'eau puis lyophilise.
Le produit brut est purifié par HPLC préparative sur colonne Kromasil (1Kg- 600 mm x 60 mm - 70ml/min) en utilisant comme phase mobile un mélange eau/acétonitrile/acide trifluoroacétique (85/15/0,1).
On lyophilise les fractions contenant le produit attendu.
*Pouvoir rotatoire:* solvant: méthanol, C=1, T=21°C, L=589nm, $[\alpha]_D$ = +32.46°.
Spectre CAD/MS/MS de $[M+H]^+$= 204,1 en accord avec la structure attendue.

**<u>EXEMPLE 2 :</u> Acide (1*R*, 2*S*)- 2-acétylthio-1-(3-aminopropyl)-cyclopentane-carboxylique, chlorhydrate**

A une solution du composé du stade H de l'exemple 1 (2,40g ; 4,72 mmoles) dans 20 mL de dichlorométhane anhydre, on ajoute goutte à goutte, à température ambiante, une solution d'HCl/dioxane 4M (35,5 mL ; 141,6 mmoles; 30eq). On abandonne le milieu réactionnel sous agitation à température ambiante jusqu'à déprotection des deux fonctions (contrôle par LC/MS - durée approximative : 1 nuit) puis on évapore les solvants. Le produit obtenu est purifié sur une colonne BIOGEL P2 en utilisant comme phase mobile un mélange eau/acétonitrile/acide chlorhydrique N (1000/1000/2). On lyophilise les fractions contenant le produit attendu.

| Microanalyse élémentaire | C | H | N | S | Cl- |
|---|---|---|---|---|---|
| Calculé % | 46.88 | 7.15 | 4.97 | 11.38 | 12.58 |
| Trouvé % | 46.82 | 7.2 | 5.23 | 10.828 | 13.13 |

**<u>EXEMPLE 3 :</u> Acide (1*R*, 2*S*)-1-(3-aminopropyl)-2-mercaptocyclopentanecarboxylique, chlorhydrate**

On porte à 45°C pendant une nuit une solution du composé de l'exemple 2 ( 4,51g ; 16 mmoles) dans 148mL d'une solution HCl 4M. Après refroidissement, on dilue le milieu réactionnel avec 150mL d'eau puis lyophilise.
Le produit brut est purifié par HPLC préparative sur colonne Kromasil (1Kg- 600 mm x 60 mm - 70ml/min) en utilisant comme phase mobile un mélange eau/acétonitrile (95/5).
On lyophilise les fractions contenant le produit attendu.

| Microanalyse élémentaire | C | H | N | S | Cl- |
|---|---|---|---|---|---|
| Calculé % | 45.09 | 7.57 | 5.84 | 13.37 | 14.79 |
| Trouvé % | 44.57 | 7.68 | 5.74 | 12.88 | 16.69 |

Spectre CAD/MS/MS de $[M+H]^+$= 204,1 en accord avec la structure attendue.

**<u>EXEMPLE 3a</u> : Acide (1R, 2S)- 1-(3-aminopropyl)-2-mercaptocyclopentane-carboxylique**

*<u>Stade A :</u> Adipate de di-tert-butyle*

A une solution de *tert*-butanol (3,27 L; 34,4 moles - 10eq), de pyridine (800 mL; 10,32 moles - 3eq) dans 3 L de toluène anhydre, on coule, goutte à goutte, à température ambiante, le chlorure d'adipoyle (629,8g ; 3,44 moles). Le milieu réactionnel est porté à 70°C, sous agitation, pendant une nuit.
Après refroidissement, le précipité de chlorure de pyridinium est filtré, rincé au toluène. La phase toluènique est lavé avec une solution d'acide chlorhydrique N (4x500mL), une solution de carbonate de sodium à 10% (1x500mL), à l'eau (1x500mL) puis avec une solution saturée de chlorure de sodium (1x500mL).
La phase organique est séchée sur sulfate de sodium, filtrée puis évaporée.
L'huile brute obtenue est purifiée par distillation fractionnée sous pression réduite (100°C / $2.10^{-2}$ mmHg).

*<u>Stade B :</u> 2-Oxo-cyclopentanecarboxylate de tert-butyle*

A une suspension de NaH (108,54g à 60%; 2,71 moles - 2,15eq) dans 1,1L de toluène anhydre, on coule, à température ambiante, du tert-butanol (5,66 mL ; 0,06 mole - 0,05eq) ainsi que 14g d'adipate de di-tert-butyle. Le milieu réactionnel est porté au reflux. A cette température, on coule, goutte à goutte, une solution d'adipate de di-tert-butyle (324,6g ; 1,26 moles) en solution dans 500mL de toluène anhydre, en contrôlant le dégagement gazeux.
Durée approximative de l'addition : 3 heures.
*$R_q$ : on note une prise en masse importante du milieu réactionnel au cours de l'addition de l'adipate de di-tert-butyle mais qui évolue vers une suspension.*
Une fois l'addition terminée, le milieu réactionnel est maintenu à reflux pendant 5 heures.
Après refroidissement, le milieu réactionnel est hydrolysé, à 0°C, avec une solution d'acide acétique à 10% ( 1L). On décante la phase organique, extrait la phase aqueuse au toluène (300ml). Les phases toluèniques regroupées sont lavées à l'eau (2x300ml), puis par une solution saturée de chlorure de sodium (300ml).
La phase organique est séchée sur sulfate de sodium, filtrée puis évaporée.

L'huile orangée est purifiée par distillation fractionnée sous pression réduite (65-70°C / 1.10$^{-1}$ mmHg).

*Stade C : (1S)-2-oxo-1-(3-oxopropyl)cyclopentanecarboxylate de tert-butyle*

A une solution de 2-oxo-cyclopentanecarboxylate de tert-butyle (5,51 g; 29,93 mmoles) et du catalyseur Q (1,46 g ; 2,99 mmoles - 0,1 eq)

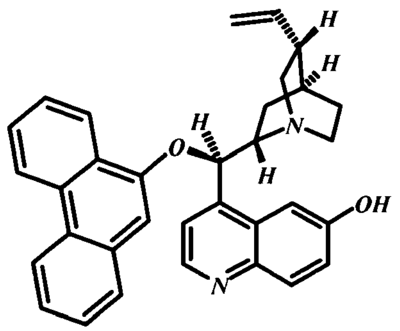

catalyseur Q - synthèse décrite dans
Angew. Chem. Int. Ed. 2005, 44, 105-108

dans 60mL de dichlorométhane anhydre, on coule en 30 minutes, à -25°C, sous argon et lente agitation, une solution d acroléïne (4,19g ; 74,83 mmoles - 2,5 eq) dans 10mL de dichlorométhane anhydre. En fin d'addition, le milieu réactionnel est maintenu sous agitation pendant quatre heures à cette température, puis une nuit à -5°C.

Le filtrat est évaporé pour conduire à une huile incolore.

*Stade D : (1S)-1-(3-hydroxypropyl)-2-oxocyclopentanecarboxylate de tert-butyle*

A une solution de cétoester aldéhyde de l'étape précédente (6,5 g; 26,9 mmoles) dans 80mL de tétrahydrofurane anhydre, on ajoute le triacétoxyborohydrure de sodium (6,84g ; 32,3 mmoles - 1,2 eq).Le milieu réactionnel est porté à reflux, sous agitation, pendant 5 heures 30. Après retour à température ambiante, le milieu réactionnel est évaporé. Le résidu est repris par 100mL de $CH_2Cl_2$, lavé par une solution saturée de bicarbonate de sodium(2x20 mL). La phase aqueuse basique est extraite au $CH_2Cl_2$. Les phases organiques regroupées sont lavées par une solution saturée de chlorure de sodium, séchées sur sulfate de sodium puis évaporées. Le produit brut est purifié par flash chromatographie sur gel de silice en utilisant comme éluant un mélange $CH_2Cl_2$/Isopropanol (95/5).

*Stade E : (1S)-1-(3-bromopropyl)-2-oxocyclopentanecarboxylate de tert-butyle*

A une solution d'alcool de l'étape précédente (5,4 g; 22,12 mmoles) et de triphénylphosphine (6,7 g; 25,44 mmoles - 1,15 eq) dans 35mL de dichlorométhane anhydre, on ajoute, le N-bromosuccinimide (4,53 g ; 25,44 mmoles - 1,15 eq) par fractions en maintenant la température à 5°C. Après 1 heure d'agitation à température ambiante, le milieu réactionnel est évaporé. Le résidu est repris par 100mL d'éther isopropylique, trituré pour obtenir la cristallisation de l'oxyde de triphénylphosphine. Le solide est filtré, rincé. Le filtrat est concentré puis repris au pentane puis à nouveau filtré. Le filtrat pentanique est évaporé. Le produit brut est filtré par flash chromatographie sur gel de silice en utilisant comme éluant un mélange Heptane/Acétate d'éthyle (8/2).

*Stade F : (1S,2R)-1-(3-bromopropyl)-2-hydroxycyclopentanecarboxylate de tert-butyle*

On coule, à température ambiante, sous agitation, une solution commerciale de *(+)DIPCl* 1.8M dans l'hexane (320mL ; 0,577 mole - 1,5eq) sur le céto-ester (117,5g ; 0,385 mole) obtenu au cours de l'étape précédente. Le milieu réactionnel est agité à 55°C une nuit à température ambiante.

On ajoute au milieu réactionnel 950mL d'éther éthylique puis sous agitation, à 0°C, l'acétaldhéhyde (35,9 mL ; 0,635 mole - 9,65eq). Le milieu réactionnel est maintenu sous agitation à température ambiante pendant 4 heures. Après refroidissement du milieu réactionnel à 10°C, on ajoute une solution de soude 6N ( 480mL ; 2,88 moles - 7,5eq). Le milieu réactionnel est maintenu sous agitation à température ambiante pendant 1 heure. La phase organique est

décantée, lavée à l'eau (3x300ml), avec une solution d'acide citrique à 10% (2x300mL) puis avec une solution saturée de chlorure de sodium( 3x150mL).La phase organique est séchée sur sulfate de sodium puis évaporée. Le produit brut est purifié par flash chromatographie sur gel de silice en utilisant comme éluant un gradient Heptane /Acétate d'éthyle (95/5 à 85/15).

*Stade G : (1S,2R)-1-{3-[bis(tert-butoxycarbonyl)amino]propyl}-hydroxycyclopentane-carboxylate de tert-butyle*

A une solution d'alcool bromé de l'étape précédente (11,8g ; 36,06 mmoles) dans 110 mL de DMF anhydre, on ajoute, à température ambiante, d'iminodicarboxylate de di-tert-butyle (7,83 g ; 36,06 mmoles; 1 eq), puis le carbonate de césium (11,75g ; 36,06 mmoles; 1eq). Le milieu réactionnel est agité à température ambiante pendant 3 heures.

Le milieu réactionnel est filtré. Après concentration du filtrat, le résidu huileux est repris par 300 mL d'acétate d'éthyle. La phase acétate d'éthyle est lavée à l'eau (3x100mL), puis par une solution saturée de chlorure de sodium (100mL). La phase organique est séchée sur sulfate de sodium, filtrée puis évaporée.

Le produit brut est purifié par flash chromatographie sur gel de silice en utilisant comme éluant un gradient Heptane /Acétate d'éthyle (9/1 à 85/15).

*Stade H : (1S,2R)-1-{3-[bis(tert-butoxycarbonyl)amino]propyl}-2-[(méthylsulfonyl)oxy]-cyclopentanecarboxylate de tert-butyle*

A une solution de l'alcool de l'étape précédente (8,70g ; 19,7 mmoles) dans 130 mL de tétrahydrofuranne anhydre, on ajoute , à température ambiante , la triéthylamine (4,11mL ; 29,55 mmoles; 1,5eq). On refroidit le milieu réactionnel à -30°C, puis coule goutte à goutte, le chlorure de mésyle (2,29 mL; 29,55 mmoles ; 1,5eq) en solution dans 10 mL de tétrahydrofuranne anhydre. On laisse remonter et agiter à température ambiante pendant deux heures. Après évaporation du tétrahydrofuranne, le milieu réactionnel est repris par 200 mL d'acétate d'éthyle. La phase acétate d'éthyle est lavée à l'eau (2x20mL) puis par une solution de chlorure de sodium saturée (1x20mL). La phase organique est séchée sur sulfate de sodium, filtrée puis évaporée.

Le produit brut est purifié par flash chromatographie sur gel de silice en utilisant comme éluant un gradient Heptane /Acétate d'éthyle (85/15 à 8/2).

*Stade I : (1R,2S)-2-(acétylthio)-{3-[bis(tert-butoxycarbonyl)amino]propyl}cyclopentanecarboxylate de tert-butyle*

Le produit attendu est obtenu à partir du composé obtenu au stade précédent selon le procédé du Stade G de l'Exemple 1.

*Stade J : Chlorhydrate de l'acide (1R,2S)-1-(3-aminopropyl)-2-mercaptocyclopentanecarboxylique*

*Déprotection des fonctions acide et amine :*

A une solution du composé obtenu dans l'étape précédente (8,80 g ; 17,6 mmoles) dans 100 mL de $CH_2Cl_2$ anhydre, on ajoute goutte à goutte, à température ambiante, une solution d'HCl/dioxane 4M (132 mL; 0,53 moles; 30 eq). On abandonne le milieu réactionnel sous agitation à température ambiante jusqu'à déprotection des deux fonctions (contrôle par LC/MS - durée approximative : 1 nuit) puis on évapore les solvants. Le produit obtenu est utilisé sans purification dans l'étape suivante.

*Déprotection du thiol :*

On reprend le produit d'évaporation par 200 mL d'une solution aqueuse HCl 4M préalablement dégazée à l'argon, puis porte à 45°C pendant une nuit. Après refroidissement, on dilue le milieu réactionnel avec 100 mL d'eau dégazée puis lyophilise.

*Stade K : Acide (1R,2S)-1-(3-aminopropyl)-2-mercaptocyclopentanecarboxylique*

Le composé du stade précédent (10g, 41.5 mmol) est dissout dans 80 mL d'eau et mis sous agitation.

La solution est jaune pâle limpide (pH environ 1.2) On ajoute sous agitation environ 11 mL d'une solution de bicarbonate de soude saturée. Dès pH=4, on observe un premier précipité, qui est filtré. Le filtrat est ensuite basifié, jusqu'à pH=5.5, en filtrant au fur et à mesure les précipités formés. Le filtrat est mis à évaporer sous pression réduite (Tbain=55°C). Lorsqu'il reste 10 mL de solution environ, on filtre à nouveau.

Les précipités de qualité équivalente sont rassemblés et séchés sous vide, pour conduire au produit du titre.

Spectroscopie RMN :

($^1$HNMR, $D_2O$, 600 MHz) δ = 3.08 ppm (1H, dd); 2.91 (2H, t); 2.18 (1H, m); 2.11 (1H, m); 1.79 (1H, m); 1.69-1.56 (4H, m); 1.52 (1H, m); 1.45 (1H, m); 1.37 (1H, m).

**EXEMPLE 3b : Acide (1*R*, *2S*)- 1-(3-aminopropyl)-2-mercaptocyclopentanecarboxylique, phosphate**

Le composé de l'Exemple 3a est mis en réaction avec l'acide phosphorique 85% dans l'eau, puis lyophilisé.

Spectroscopie RMN :

($^1$H NMR, $D_2O$, 600 MHz) δ = 3.09 ppm (1H, t); 2.93 (2H, t); 2.18 (2H, m); 1.82 (1H, m); 1.79 (1H, dt); 1.72-1.50 (5H, m); 1.41 (1H, dt).

**EXEMPLE 3c : Acide (1*R*, 2*S*)-1-(3-aminopropyl)-2-mercaptocyclopentane-carboxylique, bésylate**

Le composé de l'Exemple 3a est mis en réaction avec l'acide benzènesulfonique dans l'eau, puis lyophilisé.

Spectroscopie RMN :

($^1$H NMR, DMSO-D6, 600 MHz) δ = 12.38 ppm (1H, sl); 7.64 (3H, sl); 7.59 (2H, m); 7.32 (2H, m); 7.29 (1H, m); 3.02 (1H, tl); 2.76 (2H, m); 2.33 (1H, sl); 2.13 (2H, m); 1.84-1.65 (3H, m); 1.61 (1H, m); 1.52 (3H, m); 1.32 (1H, m).

**EXEMPLE 4 : Acide (1*S*, 2R)- 1-(3-aminopropyl)-2-mercaptocyclopentanecarboxylique, trifluoroacétate**

Le produit attendu est obtenu selon le procédé décrit aux stades I et J de l'exemple 1, à partir de l'autre énantiomère obtenu au stade H de l'exemple 1, c'est-à-dire le (1S, 2R)- 2-(acétylthio)-1-(3-{[(benzyloxy)carbonyl]amino}propyl)cyclopentane-carboxylate de benzyle.

Spectre CAD/MS/MS de $[M+H]^+$ = 204,1 en accord avec la structure attendue

**EXEMPLE 5 : Acide (1*S,* 2R)- 2-acétylthio-1-(3-aminopropyl)-cyclopentane-carboxylique, chlorhydrate**

Le produit attendu est obtenu selon le procédé décrit pour l'exemple 2, à partir de l'autre énantiomère obtenu au stade H de l'exemple 1, c'est-à-dire le (1S, 2R)- 2-(acétylthio)-1-(3-{[(benzyloxy)carbonyl]amino}propyl)cyclopentane-carboxylate de benzyle.

| Microanalyse élémentaire | C | H | N | S | Cl- |
|---|---|---|---|---|---|
| Calculé % | 46.88 | 7.15 | 4.97 | 11.38 | 12.58 |
| Trouvé % | 46.90 | 7.25 | 5.02 | 11.09 | 12.78 |

**EXEMPLE 6 : Acide 1-(3-aminopropyl)-2-mercaptocyclopentanecarboxylique, trifluoroacétate (diastéréoisomère cis racémique)**

Le produit attendu est obtenu selon le procédé décrit aux stades I et J de l'exemple 1, à partir du composé obtenu au stade G de l'exemple 1.

Spectre CAD/MS/MS de $[M+H]^+$ = 204,1 en accord avec la structure attendue.

**EXEMPLE 7 : Acide 1-(4-aminobutyl)-2-mercaptocyclopentanecarboxylique, trifluoroacétate (diastéréoisomère trans racémique)**

*Stade A : 1-(4-Bromobutyl)-2-oxo-cyclopentanecarboxylate de benzyle*

Dans un tricol de 500 mL muni d'une agitation magnétique et d'un réfrigérant, sous atmosphère inerte d'azote, l'hydrure de sodium 95% (2,2 g ; 87,1 mmol ; 1,25 éq.) est mis en suspension dans un mélange de THF anhydre (115 mL) et de HMPA (14,6 mL). La solution de 2-oxocyclopentanecarboxylate de benzyle (15,3 g ; 70,3 mmol) dans 45 mL de THF anhydre est ajoutée goutte à goutte pour maintenir la température inférieure à 45°C. Le milieu réactionnel est

agité 1 heure à température ambiante. A la solution jaune limpide, le 1,4-dibromobutane (12,6 mL ; 105,4 mmol ; 1,5 éq.) est ajoutée. Le milieu réactionnel est encore agité au reflux pendant 14 heures. Après être revenu à température ambiante, 80 mL d'une solution aqueuse saturée de chlorure d'ammonium sont ajoutés. Le mélange est versé dans 500 mL d'éther et la phase organique est lavée par 5 fois 100 mL d'eau puis 100 mL d'une solution aqueuse saturée de chlorure de sodium. La phase organique est séchée sur $MgSO_4$, filtrée et concentrée.

Le produit est purifié sur gel de silice en utilisant comme éluant un mélange cyclohexane/acétate d'éthyle (gradient 95/5 à 80/20) pour conduire au produit attendu sous forme d'une huile jaune.

*Stade B : 1-(4-Bromobutyl)-2-hydroxy-cyclopentanecarboxylate de benzyle*

Le produit attendu est obtenu selon le procédé décrit au stade E de l'exemple 1, à partir du composé obtenu au stade précédent.

*Stade C : 1-(4-Azidobutyl)-2-hydroxy-cyclopentanecarboxylate de benzyle*

Dans un ballon, sous atmosphère inerte d'azote, le composé obtenu à l'étape précédente (4,41 g ; 16,6 mmol), l'azoture de sodium (5,5 g ; 84,6 mmol ; 5 éq.) et l'iodure de sodium (0,2 g) sont mis en suspension dans 60 mL d'éthanol. Le milieu réactionnel est agité au reflux pendant 24 heures puis concentré à sec. Le résidu huileux est solubilisé dans 150 mL d'éther et 80 mL d'eau. La phase organique est lavée par 80 mL d'eau et 40 mL d'une solution aqueuse saturée de chlorure de sodium. La phase organique est séchée sur $MgSO_4$, filtrée et concentrée.

Le mélange d'azotures (Rac)-trans et (Rac)-cis formé est utilisé tel quel dans la suite de la réaction.

*Stade D : 1-(4-Aminobutyl)-2-hydroxy-cyclopentanecarboxylate de benzyle*

Dans un ballon, le mélange d'azotures (Rac)-trans et (Rac)-cis (20,64 g ; 62,3 mmol) et la triphénylphosphine (24,4 g ; 93 mmol ; 1,5 éq.) sont dissous dans 500 mL de THF. La solution est agitée 1 heure à 50°C. De l'eau (10 mL ; 556 mmol ; 9 éq.) est ajoutée. Le milieu réactionnel est agité 4 heures à 50°C puis concentré à sec. Le résidu huileux est solubilisé dans 250 mL d'éther et la phase organique est extraite par 2 fois 120 mL d'une solution aqueuse d'acide chlorhydrique 1 N. La phase aqueuse est lavée par 100 mL d'éther. Le pH de la phase aqueuse est ramené à 12-13 par ajout de potasse solide. La phase aqueuse est extraite par 3 fois 250 mL d'acétate d'éthyle. Les phases organiques réunies sont lavées par 100 mL d'eau puis 100 mL d'une solution aqueuse saturée de chlorure de sodium. La phase organique est séchée sur $MgSO_4$, filtrée et concentrée.

Le produit attendu, obtenu sous la forme d'un mélange cis/trans, est utilisé tel quel dans la suite de la réaction.

*Stade E : 1-(4-{[Benzyloxy)carbonyl]amino}butyl)-2-hydroxy-cyclopentanecarboxylate de benzyle*

Dans un ballon, le produit obtenu au stade précédent (62,3 mmol) est dissous dans 160 mL de dioxane. Une solution aqueuse de carbonate de sodium (13,16 g ; 124 mmol ; 2 éq.) dans 160 mL d'eau est ajoutée. À 0°C, ajouter lentement la solution de chloroformiate de benzyle (12 mL ; 84,4 mmol ; 1,35 éq.) dans 30 mL de dioxane. Le milieu réactionnel est agité à température ambiante pendant 12 heures puis partiellement concentré. La phase aqueuse est extraite par 2 fois 250 mL d'éther. Les phases organiques réunies sont lavées, séchées, filtrées et concentrées.

Le produit est purifié sur gel de silice (éther de pétrole/acétate d'éthyle : gradient 9/1 à 2/8) pour conduire au produit attendu sous forme d'une huile jaune.

*Stade F : 1-(4-{[Benzyloxy)carbonyl]amino}butyl)-2-{(méthylsulfonyl)oxy-cyclopentanecarboxylate de benzyle (diastéréoisomère cis racémique)*

Le produit attendu est obtenu selon le procédé décrit au stade F de l'exemple 1, à partir du composé obtenu au stade précédent.

Le diastéréoisomère cis est le premier dans l'ordre d'élution.

*Stade G : 2-(Acétylthio)-1-(4-{[benzyloxy)carbonyl]amino}butyl)-cyclopentanecarboxylate de benzyle (diastéréoisomère trans racémique)*

Le produit attendu est obtenu selon le procédé décrit au stade G de l'exemple 1, à partir du composé cis obtenu au stade précédent.

*Stade H: Acide 1-(4-aminobutyl)-2-mercaptocyclopentanecarboxylique, trifluoroacétate (diastéréoisomère trans racémique)*

Le produit attendu est obtenu selon le procédé décrit aux stades I et J de l'exemple 1, à partir du composé obtenu au stade précédent.

| Microanalyse élémentaire | C | H | N | S |
|---|---|---|---|---|
| Calculé % | 43,5 | 6,08 | 4,23 | 9,68 |
| Trouvé % | 43,9 | 6,24 | 4,22 | 9,59 |

**EXEMPLE 8 : Acide 1-(4-aminobutyl)-2-mercaptocyclopentanecarboxylique, trifluoroacétate (diastéréoisomère cis racémique)**

*Stade A : 1-(4-{[Benzyloxy)carbonyl]amino}butyl)-2-[(méthylsulfonyl)oxy-cyclopentanecarboxylate de benzyle (diastéréoisomère trans racémique)*

Le produit attendu est l'autre diastéréoisomère obtenu au stade F de l'exemple 7 (diastéréoisomère trans).

*Stade B : Acide 1-(4-aminobutyl)-2-mercaptocyclopentanecarboxylique, trifluoroacétate (diastéréoisomère cis racémique)*

Le produit attendu est obtenu selon le procédé décrit aux stades G, I et J de l'exemple 1, à partir du composé obtenu au stade précédent.

| Microanalyse élémentaire | C | H | N | S |
|---|---|---|---|---|
| Calculé % | 43,5 | 6,08 | 4,23 | 9,68 |
| Trouvé % | 43,56 | 6,26 | 4,13 | 9,36 |

**EXEMPLE 9 : Acide 1-(4-aminobutyl)-2-mercaptocyclopentanecarboxylique, trifluoroacétate (diastéréoisomère cis énantio 1)**

*Stade A : 2-(Acétylthio)-1-(4-{[benzyloxy)carbonyl]amino}butyl)-cyclopentanecarboxylate de benzyle (diastéréoisomère cis, énantio 1)*

Le produit attendu est obtenu selon le procédé décrit aux stades G et H de l'exemple 1, à partir du composé obtenu au stade A de l'exemple 8.

L'énantio 1 est le *premier* dans l'ordre d'élution.

*Stade B : Acide 1-(4-aminobutyl)-2-mercaptocyclopentanecarboxylique, trifluoroacétate (diastéréoisomère cis, énantio 1)*

Le produit attendu est obtenu selon le procédé décrit aux stades I et J de l'exemple 1, à partir du composé obtenu au stade précédent.

Spectre CAD/MS/MS de $[M+H]^+$= 218 en accord avec la structure attendue.

**EXEMPLE 10 : Acide 1-(4-aminobutyl)-2-mercaptocyclopentanecarboxylique, trifluoroacétate (diastéréoisomère cis énantio 2)**

*Stade A : 2-(Acétylthio)-1-(4-{[benzyloxy)carbonyl]amino}butyl)-cyclopentanecarboxylate de benzyle (diastéréoisomère cis, énantio 2)*

Le produit attendu est l'autre énantiomère obtenu au stade A de l'exemple 9.

*Stade B : Acide 1-(4-aminobutyl)-2-mercaptocyclopentanecarboxylique, trifluoroacétate (diastéréoisomère cis, énantio 2)*

Le produit attendu est obtenu selon le procédé décrit aux stades I et J de l'exemple 1, à partir du composé obtenu au stade précédent.
Spectre CAD/MS/MS de $[M+H]^+$ = 218 en accord avec la structure attendue

**EXEMPLE 11 : Acide 1-[(4-aminométhyl)benzyl]-2-mercaptocyclopentanecarboxylique, trifluoroacétate (diastéréoisomère cis racémique)**

*Stade A : 1-(4-Bromométhyl)benzyl]-2-oxo-cyclopentanecarboxylate de benzyle*

Le produit attendu est obtenu selon le procédé décrit au stade A de l'exemple 7, à partir de 2-oxo-cyclopentanecarboxylate de benzyle et de 1,4-dibromométhylbenzène.

*Stade B : 1-(4-Bromométhyl)benzyl]-2-hydroxy-cyclopentanecarboxylate de benzyle (diastéréoisomère trans racémique)*

Le produit attendu est obtenu selon le procédé décrit au stade E de l'exemple 1, suivi par une séparation diastéréoisomérique du composé ainsi obtenu par flash chromatographie sur gel de silice en utilisant comme éluant un mélange cyclohexane / acétate d' éthyle (gradient 90/10 à 65/35).
Le diastéréoisomère trans est le premier dans l'ordre d'élution.

*Stade C : Acide 1-[(4-aminométhyl)benzyl]-2-mercaptocyclopentanecarboxylique, trifluoroacétate (diastéréoisomère cis racémique)*

Le produit attendu est obtenu selon le procédé décrit aux stades C à H de l'exemple 7 à partir du composé obtenu au stade précédent.
Spectre CAD/MS/MS de $[M+H]^+$ = 266,1 en accord avec la structure attendue

**EXEMPLE 12 : Acide 2-mercapto-1(pipéridin-4-ylméthyl)-cyclopentane-carboxylique, trifluoroacétate (diastéréoisomère trans racémique)**

*Stade A : 4-({1-{[Benzyloxy)carbonyl]-2-hydroxy-cyclopentyl)méthyl)-1-pipéridinecarboxylate de benzyle (diastéréoisomère cis racémique)*

Le produit attendu est obtenu selon le procédé décrit aux stades A et B de l'exemple 7 à partir de 2-oxo-cyclopentanecarboxylate de benzyle et de 4-bromométhyl-1-pipéridinecarboxylate de benzyle.
Le diastéréoisomère cis est le deuxième dans l'ordre d'élution.

*Stade B : 4-({1-[(Benzyloxy)carbonyl]-2-[(méthylsulfonyl)oxy]cyclopentyl}méthyl)-1-pipéridinecarboxylate de benzyle (diastéréoisomère cis racémique)*

Le produit attendu est obtenu selon le procédé décrit au stade F de l'exemple 1, à partir du composé obtenu au stade précédent.

*Stade C : Acide 2-mercapto-1-(pipéridin-4-ylméthyl)-cyclopentane-carboxylique, trifluoroacétate (diastéréoisomère trans racémique)*

Le produit attendu est obtenu selon le procédé décrit aux stades G, I et J de l'exemple 1, à partir du composé obtenu au stade précédent.

| Microanalyse élémentaire | C | H | N | S |
|---|---|---|---|---|
| Calculé % | 47,05 | 6,2 | 3,92 | 8,97 |
| Trouvé % | 47,12 | 6,52 | 4,03 | 7,6 |

**EXEMPLE 13 :** Acide **2-mercapto-1(pipéridin-4-ylméthyl)-cyclopentane-carboxylique, trifluoroacétate** (diastéréoisomère cis racémique)

*Stade A : 4-({1-[(Benzyloxy)carbonyl]-2-[(méthylsulfonyl)oxy]cyclopentyl}méthyl)-1-pipéridinecarboxylate de benzyle (diastéréoisomère trans racémique)*

Le produit attendu est l'autre diastéréoisomère obtenu au stade B de l'exemple 12 (diastéréoisomère trans).

*Stade B : Acide 2-mercapto-1-(pipéridin-4-ylméthyl)-cyclopentane-carboxylique, trifluoroacétate (diastéréoisomère cis racémique)*

Le produit attendu est obtenu selon le procédé décrit aux stades G, I et J de l'exemple 1, à partir du composé obtenu au stade précédent.

| Microanalyse élémentaire | C | H | N | S |
|---|---|---|---|---|
| Calculé % | 47,05 | 6,2 | 3,92 | 8,97 |
| Trouvé % | 46,58 | 6,24 | 4,02 | 7,78 |

**EXEMPLE 14 : Acide 1-(5-aminopentyl)-2-mercaptocyclopentanecarboxylique, trifluoroacétate (diastéréoisomère trans racémique)**

*Stade A : 1-(5-{[Benzyloxy)carbonyl]amino}pentyl)-2-hydroxy-cyclopentanecarboxylate de benzyle*

Le produit attendu est obtenu selon le procédé décrit aux stades A à E de l'exemple 7 à partir de 2-oxo-cyclopentanecarboxylate de benzyle et de 1,5-dibromopentane.

*Stade B : 1-(5-{[Benzyloxy)carbonyl]amino}pentyl)-2-[(méthylsulfonyl)oxy-cyclopentanecarboxylate de benzyle (diastéréoisomère cis racémique)*

Le produit attendu est obtenu selon le procédé décrit au stade F de l'exemple 1, à partir du composé obtenu au stade précédent.

Le diastéréoisomère cis est le *deuxième* dans l'ordre d'élution.

*Stade C : Acide 1-(5-aminopentyl)-2-mercaptocyclopentanecarboxylique, trifluoroacétate (diastéréoisomère trans racémique)*

Le produit attendu est obtenu selon le procédé décrit aux stades G, I et J de l'exemple 1, à partir du composé obtenu au stade précédent.

Spectre CAD/MS/MS de $[M+H]^+$= 232,1 en accord avec la structure attendue

**EXEMPLE 15 : Acide 1-(5-aminopentyl)-2-mercaptocyclopentanecarboxylique, trifluoroacétate (diastéréoisomère cis racémique)**

*Stade A : 1-(5-{[Benzyloxy)carbonyl]amino}pentyl)-2-[(méthylsulfonyl)oxy-cyclopentanecarboxylate de benzyle (diastéréoisomère trans racémique)*

Le produit attendu est l'autre diastéréoisomère obtenu au stade B de l'exemple 14 (diastéréoisomère trans).

*Stade B : Acide 1-(5-aminopentyl)-2-mercaptocyclopentanecarboxylique, trifluoroacétate (diastéréoisomère cis racémique)*

Le produit attendu est obtenu selon le procédé décrit aux stades G, I et J de l'exemple 1, à partir du composé obtenu au stade précédent.

| Microanalyse élémentaire | C | H | N | S |
|---|---|---|---|---|
| Calculé % | 45,21 | 6,42 | 4,06 | 9,28 |
| Trouvé % | 45,41 | 6,76 | 4,1 | 9,75 |

**<u>EXEMPLE 16</u> : Acide 2-mercapto-1-(2-pipéridin-4-yléthyl)-cyclopentane-carboxylique, trifluoroacétate (diastéréoisomère cis racémique)**

*<u>Stade A</u> : 4-(2-{1-[(Benzyloxy)carbonyl]-2-hydroxy-cyclopentyl)éthyl)-1-pipéridinecarboxylate de benzyle*

Le produit attendu est obtenu selon le procédé décrit aux stades A et B de l'exemple 7 à partir de 2-oxocyclopentanecarboxylate de benzyle et de 4-(2-bromoéthyl)-1-pipéridinecarboxylate de benzyle.

Le diastéréoisomère trans est le premier dans l'ordre d'élution.

*<u>Stade B :</u> 4-(2-{1-[(Benzyloxy)carbonyl}-2-[(méthylsulfonyl)oxy]cyclopentyl)éthyl)-1-pipéridinecarboxylate de benzyle (diastéréoisomère trans racémique)*

Le produit attendu est obtenu selon le procédé décrit au stade F de l'exemple 1, à partir du composé obtenu au stade précédent.

*<u>Stade C :</u> Acide 2-mercapto-1-(2-pipéridin-4-yléthyl)-cyclopentane-carboxylique, trifluoroacétate (diastéréoisomère cis racémique)*

Le produit attendu est obtenu selon le procédé décrit aux stades G, I et J de l'exemple 1, à partir du composé obtenu au stade précédent.

Spectre CAD/MS/MS de $[M+H]^+$= 258 en accord avec la structure attendue.

**<u>EXEMPLE 17</u> : Acide 2-mercapto-1-(pyridin-3-ylméthyl)-cyclopentane-carboxylique, trifluoroacétate (diastéréoisomère cis racémique)**

*<u>Stade A</u> : 2-Oxo-1-(3-pyridylméthyl)cyclopentanecarboxylate de benzyle*

Dans un tricol de 1L muni d'une agitation magnétique et d'un réfrigérant, sous atmosphère inerte d'azote, l'hydrure de potassium 32% (25 g ; 200 mmoles ; 1,1 éq.) est mis en suspension dans le THF anhydre (200 mL). On refroidit le milieu réactionnel à -78°C puis le 2-oxocyclopentanecarboxylate de benzyle (39,7 g ; 182 mmoles) est ajouté goutte à goutte en maintenant la température inférieure à -78°C. Le milieu réactionnel est agité 1 heure à température ambiante. A la solution jaune limpide, une solution de 3-(chlorométhyl)-pyridine base (38,7g ; 226 mmoles ; 1,24 éq.) dans 100 mL de THF anhydre est ajoutée. Le milieu réactionnel est agité au reflux pendant 12 heures. Après être revenu à température ambiante, on évapore le milieu réactionnel à sec reprend par 80 mL d'eau et 500mL d'acétate d'éthyle. La phase aqueuse est extraite par 3 fois 50 mL d' d'acétate d'éthyle. Les phases organiques réunies sont lavées par une solution saturée de chlorure de sodium puis séchées sur $MgSO_4$, filtrées et concentrées.

Le produit est purifié sur gel de silice en utilisant comme éluant un mélange dichlorométhane/éthanol (gradient 98/2 à 95/5) pour conduire au produit attendu sous forme d'une huile jaune.

*<u>Stade B</u> : 2-Hydroxy-1-(3-pyridylméthyl)cyclopentanecarboxylate de benzyle (diastéréoisomère trans racémique)*

Le produit attendu est obtenu selon le procédé décrit au stade E de l'exemple 1.

Le produit est purifié sur gel de silice en utilisant comme éluant un mélange dichlorométhane/acétate d'éthyle (gradient 7/3 à 5/5) pour conduire au produit attendu sous forme d'une huile incolore.

Le diastéréoisomère trans est le deuxième dans l'ordre d'élution.

*<u>Stade C:</u> Acide 2-mercapto-1-(pyridin-3-ylméthyl)-cyclopentane-carboxylique, trifluoroacétate (diastéréoisomère cis racémique)*

Le produit attendu est obtenu selon le procédé décrit aux stades F,G, I et J de l'exemple 1, à partir du composé obtenu au stade précédent.

| Microanalyse élémentaire | C | H | N | S |
|---|---|---|---|---|
| Calculé % | 47,86 | 4,59 | 3,99 | 9,13 |
| Trouvé % | 45,85 | 4,93 | 3,85 | 7,26 |

Spectre CAD/MS/MS de $[M+H]^+$ = 238,1 en accord avec la structure attendue

**EXEMPLE 18 : Acide 2-mercapto-1-(2-pyridin-2-yléthyl)-cyclopentane-carboxylique, trifluoroacétate (diastéréoisomère cis racémique)**

*Stade A : 2-Oxo-1-(2-pyridin-2-yléthyl)cyclopentanecarboxylate de benzyle*

Le produit attendu est obtenu selon le procédé décrit au stade A de l'exemple 17, à partir de 2-oxo-cyclopentanecarboxylate de benzyle et de 2-pyridin-2-yléthanyl méthane sulfonate.

*Stade B : 2-Hydroxy-1-(2-pyridin-2-yléthyl)cyclopentanecarboxylate de benzyle (diastéréoisomère trans racémique)*

Le produit attendu est obtenu selon le procédé décrit au stade B de l'exemple 17.
Le diastéréoisomère trans est le deuxième dans l'ordre d'élution.

*Stade C : Acide 2-mercapto-1-(2-pyridin-2-yléthyl)-cyclopentane-carboxylique, trifluoroacétate (diastéréoisomère cis racémique)*

Le produit attendu est obtenu selon le procédé décrit aux stades F,G, I et J de l'exemple 1, à partir du composé obtenu au stade précédent.

| Microanalyse élémentaire | C | H | N | S |
|---|---|---|---|---|
| Calculé % | 49,31 | 4,97 | 3,83 | 8,78 |
| Trouvé % | 45,54 | 5,11 | 3,48 | 7,56 |

Spectre CAD/MS/MS de $[M+H]^+$= 252,1 en accord avec la structure attendue

**EXEMPLE 19 : Acide 1-[(6-aminopyridin-3-ylméthyl)]-2-mercaptocyclopentane-carboxylique, trifluoroacétate (diastéréoisomère cis racémique)**

*Stade A : 2-Oxo-cyclopentanecarboxylate de tert-butyle*

1) Adipate de di-tert-butyle :

A une solution de tert-butanol (3,27 L; 34,4 moles - 10eq), de pyridine (800 mL; 10,32 moles - 3eq) dans 3 L de toluène anhydre, on coule, goutte à goutte, à température ambiante, le chlorure d'adipoyle (629,8g ; 3,44 moles). Le milieu réactionnel est porté à 70°C, sous agitation, pendant une nuit.
Après refroidissement, le précipité de chlorure de pyridinium est filtré, rincé au toluène. La phase toluènique est lavé avec une solution d'acide chlorhydrique N (4x500mL), une solution de carbonate de sodium à 10% (1x500mL), à l'eau (1x500mL) puis avec une solution saturée de chlorure de sodium (1x500mL).
La phase organique est séchée, filtrée puis évaporée.
L'huile brute obtenue est purifiée par distillation fractionnée sous pression réduite (100°C / $2.10^{-2}$ mmHg).

2) 2-Oxo-cyclopentanecarboxylate de tert-butyle

A une suspension de NaH (108,54g à 60%; 2,71 moles - 2,15eq) dans 1,1L de toluène anhydre, on coule, à température ambiante, du tert-butanol (5,66 mL ; 0,06 mole - 0,05eq) ainsi que 14g d'adipate de di-tert-butyle. Le milieu réactionnel est porté au reflux. A cette température, on coule, goutte à goutte, une solution d'adipate de di-tert-butyle (324,6g ; 1,26 moles) en solution dans 500mL de toluène anhydre, en contrôlant le dégagement gazeux.

Durée approximative de l'addition : 3 heures.

Rq : on note une prise en masse importante du milieu réactionnel au cours de l'addition de l'adipate de di-tert-butyle mais qui évolue vers une suspension.

Une fois l'addition terminée, le milieu réactionnel est maintenu à reflux pendant 5 heures.

Après refroidissement, le milieu réactionnel est hydrolysé, à 0°C, avec une solution d'acide acétique à 10% (1L). On décante la phase organique, extrait la phase aqueuse au toluène (300ml). Les phases toluéniques regroupées sont lavées à l'eau (2x300ml), puis par une solution saturée de chlorure de sodium (300ml).

La phase organique est séchée sur sulfate de sodium, filtrée puis évaporée.

L'huile orangée est purifiée par distillation fractionnée sous pression réduite (65-70°C / $1.10^{-1}$ mmHg).

*<u>Stade B :</u> 1-({6-[(tert-Butoxycarbonyl)amino]-3-pyridinyl}méthyl)-2-oxocyclopentane-carboxylate de tert-butyle*

Le produit attendu est obtenu selon le procédé décrit au stade A de l'exemple 17, à partir de 2-oxo-cyclopentanecarboxylate de tert-butyle et de [5-(chlorométhyl)pyridin-2-yl]carbamate de tert-butyle.

*<u>Stade C</u> : 1-({6-[(tert-Butoxycarbonyl)amino]-3-pyridinyl}méthyl)-2-hydroxy-cyclopentanecarboxylate de tert-butyle*

Le produit attendu est obtenu selon le procédé décrit au stade B de l'exemple 17.

Le produit est purifié sur gel de silice en utilisant comme éluant un mélange dichlorométhane/éthanol (gradient 98/2 à 95/5) pour conduire au produit attendu sous forme d'une huile incolore.

Le diastéréoisomère trans est le deuxième dans l'ordre d'élution.

*<u>Stade D :</u> 1-({6-[(tert-Butoxycarbonyl)amino]-3-pyridinyl}méthyl)-2-[(méthylsulfonyl)oxy]-cyclopentanecarboxylate de tert-butyle (diastéréoisomère trans racémique)*

Le produit attendu est obtenu selon le procédé décrit au stade F de l'exemple 1, à partir du composé obtenu au stade précédent.

*<u>Stade E :</u> Acide 1-[(6-aminopyridin-3-ylméthyl)]-2-(acéthylthio)-cyclopentane-carboxylique (diastéréoisomère cis racémique)*

Le produit attendu est obtenu selon le procédé décrit au stade G de l'exemple 1, à partir du composé obtenu au stade précédent.

*<u>Stade F :</u> Acide 1-[(6-aminopyridin-3-ylméthyl)]-2-mercaptocyclopentane-carboxylique, trifluoroacétate (diastéréoisomère cis racémique)*

<u>Déprotection des fonctions acide et amine :</u>

A une solution du composé obtenu dans l'étape précédente (2,40g ; 4,72 mmoles) dans 20 mL de $CH_2Cl_2$ anhydre, on ajoute goutte à goutte, à température ambiante, une solution d'HCl/Dioxane 4M (35,5 mL ; 141,6 mmoles; 30eq). On abandonne le milieu réactionnel sous agitation à température ambiante jusqu'à déprotection des deux fonctions (amine et acide - contrôle par LC/MS - durée approximative : 1 nuit) puis on évapore les solvants.

<u>Déprotection du thiol :</u>

On reprend le produit d'évaporation par une solution aqueuse HCl 4M, puis porte à 45°C pendant une nuit. On dilue le milieu réactionnel avec de l'eau puis lyophilise.

Le produit est purifié sur BIOGEL P2 en utilisant comme éluant un mélange eau/acétonitrile (1/1). On lyophilise les fractions contenant le produit attendu.

Spectre CAD/MS/MS de $[M+H]^+$= 253,1 en accord avec la structure attendue

**<u>EXEMPLE 20</u> : Acide (1R, 2S, l'R, 2'S)- 2,2'-disulfanediylbis[1-(3-aminopropyl) cyclopentanecarboxylique], dichlorhydrate**

On porte à 65°C pendant une nuit, une solution du composé décrit au stade J de l'exemple 1 (0,44g ;1,39 mmoles) dans 3 mL d'hydroxyde de sodium N. Après refroidissement, on neutralise le milieu réactionnel par 3 mL d'une solution d'acide chlorhydrique N.

Le produit est purifié sur BIOGEL P2 en utilisant comme éluant un mélange eau/acétonitrile (1/1). On lyophilise les fractions contenant le produit attendu.

| Microanalyse élémentaire | C | H | N | S | Cl- |
|---|---|---|---|---|---|
| Calculé % | 45.28 | 7.18 | 5.87 | 13.43 | 14.85 |
| Trouvé % | 45.31 | 6.83 | 5.94 | 13.32 | 14.54 |

**EXEMPLE 21 : Acide (1R, 2S)- 1-[3-(diméthylamino)propyl]-2-mercaptocyclopentane-carboxylique, trifluoroacétate**

*Stade A : Acide (1R, 2S, 1'R, 2'S)- 2,2'-disulfanediylbis[1-(3-(diméthylamino)pyopyl) cyclopentanecarboxylique*

A une solution du composé obtenu dans l'exemple 20 (0,57g; 0,70 mmoles) dans 4,5 mL d'acide formique, on ajoute goutte à goutte, à température ambiante, 0,2 mL d'une solution aqueuse à 37% de formaldéhyde. On porte le milieu réactionnel sous agitation à reflux pendant une heure. Après refroidissement, on dilue le milieu réactionnel avec 10 mL d'eau puis on évapore les solvants. Le produit est utilisé sans purification dans l'étape suivante.

*Stade B : Acide (1R, 2S)- 1-[3-(diméthylamino)propyl]-2-mercaptocyclopentane-carboxylique, trifluoroacétate*

A une solution du composé obtenu dans l'étape précédente dans 35 mL d'un mélange eau/THF(1/1), on ajoute goutte à goutte, à température ambiante, 2 mL de tributylphosphine. On porte le milieu réactionnel sous agitation à 50°C pendant une nuit. Après refroidissement, on dilue le milieu réactionnel avec 100 mL d'eau puis lave la phase aqueuse avec de l'éther (3x25mL). On évapore la phase aqueuse.

Le produit brut est purifié par HPLC préparative sur colonne Kromasil (1Kg- 600 mm x 60 mm - 70ml/min) en utilisant comme phase mobile un mélange eau/acétonitrile/acide trifluoroacétique (85/15/0,1).

On lyophilise les fractions contenant le produit attendu.

Spectre CAD/MS/MS de $[M+H]^+$ = 232,1 en accord avec la structure attendue

**EXEMPLE 22 : Acide 1-[3-(méthylamino)propyl]-2-mercaptocyclopentane-carboxylique, trifluoroacétate (diastéréoisomère cis racémique)**

*Stade A : 2-[(Méthylsulfonyl)oxy]-1-(3-pyridylméthyl)cyclopentanecarboxylate de benzyle (diastéréoisomère trans racémique)*

Le produit attendu est obtenu selon le procédé décrit aux stades A,B et C de l'exemple 17, à partir de 2-oxo-cyclopentanecarboxylate de benzyle et de 3-[[(benzyloxy)carbonyl](méthyl)amino]propyl méthanesulfonate.

Le diastéréoisomère trans est le deuxième dans l'ordre d'élution.

*Stade B : Acide 1-[3-(méthylamino)propyl]-2-mercaptocyclopentane-carboxylique, trifluoroacétate (diastéréoisomère cis racémique)*

Le produit attendu est obtenu selon le procédé décrit aux stades G, I et J de l'exemple 1, à partir du composé obtenu au stade précédent.

Spectre CAD/MS/MS de $[M+H]^+$ = 218,1 en accord avec la structure attendue

**EXEMPLE 23 : Acide 2-mercapto-1-(azétidin-3-ylméthyl)-cyclopentane-carboxylique, trifluoroacétate (diastéréoisomère cis racémique)**

*Stade A : 3-({1-(tert-Butyloxycarbonyl)-2-hydroxy-cyclopentyl}méthyl)-1-azétidine carboxylate de benzyle*

Le produit attendu est obtenu selon le procédé décrit aux stades A et B de l'exemple 7 à partir de 2-oxo-cyclopentanecarboxylate de benzyle et de 3-{[(méthylsulfonyl)oxy]méthyl}azétidine-1-carbamate de tert-butyle.

*Stade B : 3-([1-(tert-Butyloxycarbonyl)-2-[(méthylsulfonyl)oxy]cyclopentyl}méthyl)-1-azétidinecarboxylate de benzyle (diastéréoisomère trans racémique)*

Le produit attendu est obtenu selon le procédé décrit au stade D de l'exemple 19, à partir du composé obtenu au stade précédent.
Le produit est purifié sur gel de silice en utilisant comme éluant un mélange dichlorométhane/acétate d'éthyle (gradient 9/1 à 8/2) pour conduire au produit attendu sous forme d'une huile incolore.
Le diastéréoisomère trans est le deuxième dans l'ordre d'élution.

*Stade C : Acide 2-mercapto-1-(azétidin-3-ylméthyl)-cyclopentane-carboxylique, trifluoroacétate (diastéréoisomère cis racémique)*

Le produit attendu est obtenu selon le procédé décrit au stade F de l'exemple 19, à partir du composé obtenu au stade précédent.
Spectre CAD/MS/MS de $[M+H]^+$ = 216,1 en accord avec la structure attendue

**ETUDE PHARMACOLOGIQUE**

**EXEMPLE 24 : Inhibition de TAFIa**

Le TAFI humain purifié (25 ng) est activé par ajout du complexe thrombine-thrombomoduline en présence de chlorure de calcium. La réaction est arrêtée après 20 minutes d'incubation à 20°C par l'ajout de l'inhibiteur irréversible de la thrombine PPACK (TAFI Activity Kit, American Diagnostica).
Le composé à tester est ajouté à la solution de TAFIa (3.2 nM) et incubé pendant 5 minutes à 20°C. Un substrat chromogène du TAFIa est ajouté puis incubé pendant 30 minutes à 37°C. La réaction enzymatique est stoppée par l'ajout d'acide sulfurique (TAFI Activity Kit, American Diagnostica). La densité optique (DO) de la solution est mesurée à 490 nm à l'aide d'un spectrophotomètre (Spectramax, Molecular Devices). La valeur de DO d'un puit contenant les réactifs sans le TAFI est soustraite de toutes les valeurs de DO mesurées. Le pourcentage d'inhibition du TAFIa à une concentration donnée du composé à tester est déterminée à l'aide de la formule suivante :

$$\% \text{ inhibition} = 100 - [(\text{DO composé x100})/\text{DO véhicule}]$$

La concentration du composé de l'invention qui inhibe 50% de l'activité enzymatique du TAFIa ($IC_{50}$) calculée à partir des pourcentages d'inhibition des valeurs de DO mesurées pour des concentrations croissantes du composé à tester à l'aide d'une régression non linéaire selon une équation quatre paramètres sigmoïdale (effet-dose). Les $IC_{50}$ obtenues avec les composés représentatifs de l'invention sont rapportées dans le tableau ci-dessous en nM :

| Composé | $IC_{50}$ (nM) |
|---|---|
| Exemple 1 | 6.7 ± 0.6 |
| Exemple 2 | 22 ± 6 |
| Exemple 3 | 7.4 ± 1.1 |
| Exemple 6 | 14 ± 2 |
| Exemple 8 | 178 ± 11 |
| Exemple 10 | 81 ± 16 |
| Exemple 13 | 184 ± 22 |
| Exemple 20 | 186 ± 26 |

**EXEMPLE 25 : Composition pharmaceutique -comprimé**

Formule de préparation pour 1000 comprimés dosés à 10 mg :

| | |
|---|---|
| Composé de l'un des Exemples 1 à 23 | 10 g |
| Hydroxypropylcellulose | 2 g |
| Amidon de blé | 10 g |
| Lactose | 100 g |
| Stéarate de magnésium | 3 g |
| Talc | 3 g |

**EXEMPLE 26 : Composition pharmaceutique -comprimé en association avec la warfarine**

| Formule de préparation pour 1000 comprimés dosés à 10 mg : | |
|---|---|
| Composé de l'un des Exemples 1 à 23 | 10 g |
| Warfarine | 2 g |
| Hydroxypropylcellulose | 2 g |
| Amidon de blé | 10 g |
| Lactose | 100 g |
| Stéarate de magnésium | 3 g |
| Talc | 3 g |

**EXEMPLE 27 : Composition pharmaceutique -comprimé en association avec l'aspirine**

| Formule de préparation pour 1000 comprimés dosés à 10 mg : | |
|---|---|
| Composé de l'un des Exemples 1 à 23 | 10 g |
| Aspirine | 100 g |
| Hydroxypropylcellulose | 2 g |
| Amidon de blé | 10 g |
| Lactose | 100 g |
| Stéarate de magnésium | 3 g |
| Talc | 3 g |

**EXEMPLE 28 : Solution injectable en association avec l'altéplase**

| Formule de préparation pour 10 ml de solution : | |
|---|---|
| Composé de l'un des Exemples 1 à 23 | 20 mg |
| Altéplase | 10 mg |
| L-Arginine | 350 µg |
| Polysorbate 80 | 1 mg |
| Acide phosphorique | qsp pH7 |
| Eau ppi | 10 ml |

**Revendications**

**1.** Composé de formule (I) :

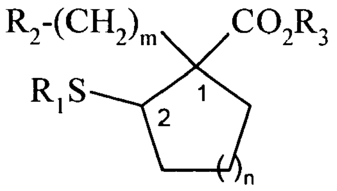

(I),

dans laquelle :

$R_1$ représente un atome d'hydrogène, un groupement de formule $COR_4$ dans laquelle $R_4$ représente un groupement alkyle $C_1$-$C_6$ linéaire ou ramifié ou un groupement aryle,
ou bien $R_1$ représente un groupement de formule (A) :

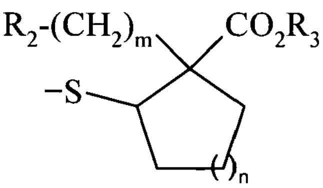

(A),

$R_2$ représente un groupement de formule $NR_5R_6$ dans laquelle $R_5$ et $R_6$, identiques ou différents, représentent chacun un atome d'hydrogène ou un groupement alkyle $C_1$-$C_6$ linéaire ou ramifié, ou bien $R_2$ représente un groupement hétérocyclique azoté, aryle ou hétéroaryle,
$R_3$ représente un atome d'hydrogène ou un groupement alkyle $C_1$-$C_6$ linéaire ou ramifié,
m représente un entier compris entre 1 et 6,
n représente 0, 1 ou 2,
ses isomères optiques, ainsi que ses sels d'addition à un acide pharmaceutiquement acceptable.

2. Composé de formule (I) selon la revendication 1, dans laquelle $R_1$ représente un atome d'hydrogène.

3. Composé de formule (I) selon la revendication 1 ou 2, dans laquelle $R_2$ représente un groupement amino ou pyridyle.

4. Composé de formule (I) selon l'une quelconque des revendications 1 à 3, dans laquelle $R_3$ représente un atome d'hydrogène.

5. Composé de formule (I) selon l'une quelconque des revendications 1 à 4, dans laquelle m représente 3.

6. Composé de formule (I) selon l'une quelconque des revendications 1 à 5, dans laquelle n représente 1.

7. Composé de formule (Ia) selon la revendication 1 :

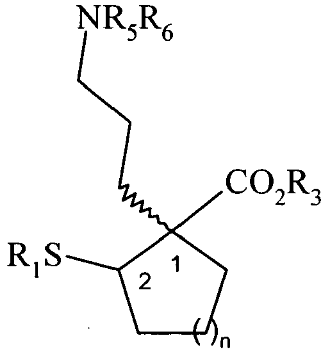

(Ia),

dans laquelle n, $R_1$, $R_3$ $R_5$ et $R_6$ sont tels que définis dans la revendication 1.

**8.** Composé de formule (I) selon la revendication 1, choisi parmi :

- l'acide (1*R*, 2*S*)-1-(3-aminopropyl)- 2-mercapto cyclopentanecarboxylique, ainsi que ses isomères optiques et ses sels d'addition à un acide pharmaceutiquement acceptable, et
- l'acide (1*R*, 2*S*)- 2-acétylthio-1-(3-aminopropyl)-cyclopentanecarboxylique, ainsi que ses isomères optiques et ses sels d'addition à un acide pharmaceutiquement acceptable.

**9.** Procédé de synthèse des composés de formule (I) selon la revendication 1 à partir du composé de formule (II) :

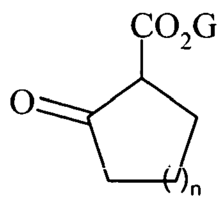

(II),

dans laquelle n représente 0, 1 ou 2, et G représente un groupement protecteur de la fonction carboxy, que l'on met en réaction avec un composé de formule (III) :

$X\text{-}(CH_2)_m\text{-}R'_2$ (III)

dans laquelle X représente un atome d'halogène ou un groupement triflate, tosylate ou mésylate, m représente un entier compris entre 1 et 6,
et $R'_2$ représente un groupement de formule $NR'_5R'_6$ dans laquelle $R'_5$ et $R'_6$, identiques ou différents, représentent chacun un groupement protecteur de la fonction amino ou un groupement alkyle $C_1$-$C_6$ linéaire ou ramifié, ou bien $R'_2$ représente un groupement hétérocyclique azoté éventuellement substitué par un groupement protecteur de la fonction amino ou bien $R'_2$ représente un groupement aryle ou hétéroaryle,
pour conduire au composé de formule (IV) :

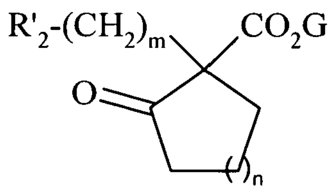

(IV),

dans laquelle $R'_2$, G, m et n sont tels que définis précédemment,
que l'on soumet à un agent réducteur de la fonction oxo, pour conduire à un composé de formule (V) :

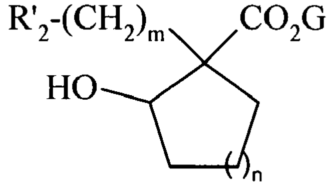

(V),

dans laquelle $R'_2$, G, m et n sont tels que définis précédemment,
que l'on met en réaction avec du chlorure de mésyle, du chlorure de tosyle, de l'anhydride triflique ou un réactif d'halogénation, pour conduire au composé de formule (VI) :

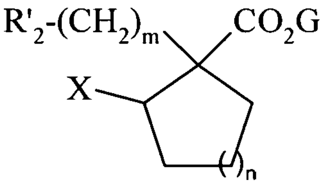

(VI),

dans laquelle R'$_2$, G, m et n sont tels que définis précédemment, et X représente un groupement mésylate, tosylate, triflate ou un atome d'halogène,
dont on sépare les diastéréoisomères, lorsque l'on souhaite obtenir un composé de formule (I) sous la forme d'un seul diastéréoisomère,
et que l'on fait ensuite réagir avec un composé de formule (VII) :

$$MS\text{-}(CO)\text{-}R'_1 \qquad (VII)$$

dans laquelle M représente le potassium, le sodium ou le lithium, et R'$_1$ représente un groupement alkyle ou aryle, pour conduire au composé de formule (VIII) :

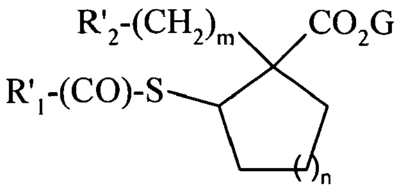

(VIII),

dans laquelle R'$_1$, R'$_2$, G, m et n sont tels que définis précédemment,
dont on sépare les énantiomères par chromatographie chirale, lorsque l'on souhaite obtenir un composé de formule (I) sous la forme d'un seul énantiomère,
et dont on déprotège le cas échéant les fonctions thiol, amino et carboxy, pour conduire au composé de formule (I), que l'on met en réaction, lorsqu'on souhaite obtenir un sel d'addition du composé de formule (I) à un acide pharmaceutiquement acceptable, avec l'acide correspondant.

**10.** Procédé de préparation des composés de formule (Ia) selon la revendication 7,
à partir du composé de formule (II) :

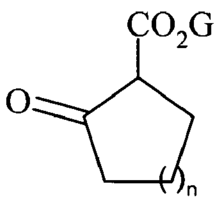

(II),

dans laquelle n représente 0, 1 ou 2, et G représente un groupement protecteur de la fonction carboxy,
que l'on met en réaction avec l'acroléïne,
en présence d'un catalyseur asymétrique,
pour conduire au composé de formule (IX), de configuration (1R) ou (1S) :

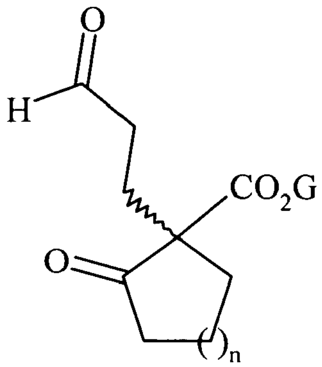

(IX),

dans laquelle n et G sont tels que définis précédemment,
dont on réduit la fonction aldéhyde, pour conduire au composé de formule (X) :

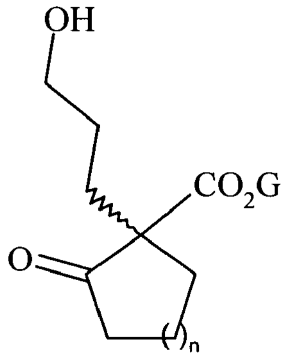

(X),

dans laquelle n et G sont tels que définis précédemment,
que l'on met en réaction avec du chlorure de mésyle, du chlorure de tosyle, de l'anhydride triflique ou un réactif d'halogénation, pour conduire au composé de formule (XI) :

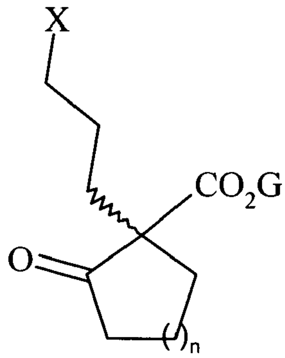

(XI),

dans laquelle n et G sont tels que définis précédemment, et X représente un atome d'halogène ou un groupement triflate, tosylate ou mésylate,
dont on réduit la fonction cétone à l'aide d'un agent réducteur, pour conduire au composé de formule (XII) :

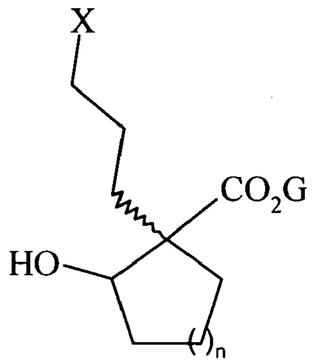

(XII),

dans laquelle n, G et X sont tels que définis précédemment, que l'on met en réaction avec un composé de formule (XIII) :

$NHR''_5R''_6$ (XIII)

dans laquelle $R''_5$ et $R''_6$ représentent chacun un groupement protecteur de la fonction amino ou un groupement alkyle $C_1$-$C_6$ linéaire ou ramifié, pour conduire au composé de formule (XIV) :

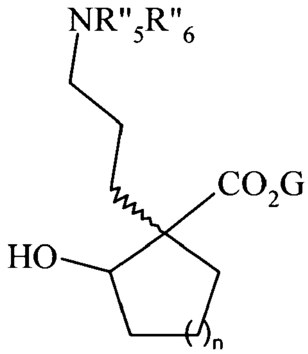

(XIV),

dans laquelle n, G, $R''_5$ et $R''_6$ sont tels que définis précédemment, que l'on met en réaction avec du chlorure de mésyle, du chlorure de tosyle, de l'anhydride triflique ou un réactif d'halogénation, pour conduire au composé de formule (XV) :

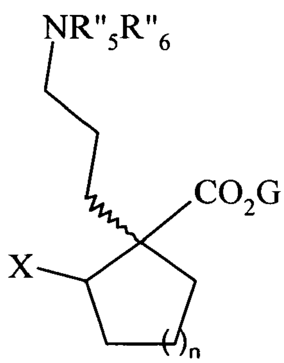

(XV),

dans laquelle n, G, $R''_5$ et $R''_6$ sont teis que définis précédemment, et X représente un groupement mésylate, tosylate, triflate ou un atome d'halogène, et que l'on fait ensuite réagir avec un composé de formule (VII) :

MS-(CO)-R'$_1$ (VII)

dans laquelle M représente le potassium, le sodium ou le lithium, et R'$_1$ représente un groupement alkyle ou aryle, pour conduire au composé de formule (XVI) :

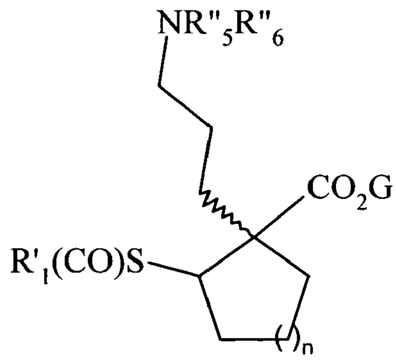

(XVI),

dans laquelle n, R'$_1$, G, R"$_5$ et R"$_6$ sont tels que définis précédemment,
dont on déprotège le cas échéant les fonctions thiol, amino et carboxy, pour conduire aux composés de formule (Ia), que l'on met en réaction, lorsqu'on souhaite obtenir un sel d'addition du composé de formule (Ia) à un acide pharmaceutiquement acceptable, avec l'acide correspondant.

**11.** Composition pharmaceutique contenant un composé de formule (I) selon l'une quelconque des revendications 1 à 8, en combinaison avec un ou plusieurs excipients ou véhicules inertes, non toxiques, pharmaceutiquement acceptables.

**12.** Composition pharmaceutique selon la revendication 11, **caractérisée en ce qu'**elle contient en outre un fibrinolytique, un anticoagulant ou un antiplaquettaire.

**13.** Composition pharmaceutique selon la revendication 11 sous forme injectable, **caractérisée en ce qu'**elle contient en outre un fibrinolytique choisi parmi le tPA recombinant, l'uPA recombinant et la streptokinase.

**14.** Composé selon l'une quelconque des revendications 1 à 8 pour son utilisation dans la prévention, la prévention secondaire ou le traitement de l'infarctus du myocarde, de l'angine de poitrine, de l'artérite des membres inférieurs, des thromboses veineuses, de l'embolie pulmonaire, des accidents vasculaires cérébraux, des anévrismes aortiques ou des démences.

**15.** Composé selon l'une quelconque des revendications 1 à 8 pour son utilisation dans la prévention, la prévention secondaire ou le traitement de l'infarctus du myocarde, de l'angine de poitrine, de l'artérite des membres inférieurs, des thromboses veineuses, de l'embolie pulmonaire, des accidents vasculaires cérébraux, des complications vasculaires du diabète, des anévrismes aortiques ou des démences, en association avec un fibrinolytique, un anticoagulant ou un antiplaquettaire.

Europäisches Patentamt
European Patent Office
Office européen des brevets

# RAPPORT DE RECHERCHE EUROPEENNE

Numéro de la demande
EP 10 29 0344

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | DO Y H ET AL: "Inhibition of thrombin activatable fibrinolysis inhibitor by cysteine derivatives" THROMBOSIS RESEARCH, TARRYTOWN, NY, US, vol. 116, no. 3, 1 janvier 2005 (2005-01-01), pages 265-271, XP004921688 ISSN: 0049-3848 * le document en entier * ----- | 1-15 | INV. C07C323/61 C07C327/34 C07D205/04 C07D211/34 C07D213/55 C07D213/73 A61P9/00 A61K31/095 A61P7/02 |
| A | WO 03/080631 A (SCHERING AG [DE]; BUCKMAN BRAD O [US]; EMAYAN KUMAR [US]; ISLAM IMADUL) 2 octobre 2003 (2003-10-02) * page 43, ligne 18 - page 46, ligne 2 * * page 67, ligne 30 - page 69, ligne 4 * * page 92 - page 94; exemples 17-19 * ----- | 1-15 | |

DOMAINES TECHNIQUES RECHERCHES (IPC)
C07C
C07D
A61P

1

Le présent rapport a été établi pour toutes les revendications

| Lieu de la recherche | Date d'achèvement de la recherche | Examinateur |
|---|---|---|
| Munich | 20 octobre 2010 | Bedel, Christian |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 10 29 0344

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-10-2010

| Document brevet cité au rapport de recherche | Date de publication | Membre(s) de la famille de brevet(s) | Date de publication |
|---|---|---|---|
| WO 03080631 A | 02-10-2003 | AU 2003230698 A1 | 08-10-2003 |
| | | BR 0308601 A | 09-01-2007 |
| | | CA 2479892 A1 | 02-10-2003 |
| | | CN 1656107 A | 17-08-2005 |
| | | EC SP045372 A | 26-11-2004 |
| | | EP 1497300 A2 | 19-01-2005 |
| | | EP 2204373 A1 | 07-07-2010 |
| | | JP 4472997 B2 | 02-06-2010 |
| | | JP 2006504622 T | 09-02-2006 |
| | | JP 2010001298 A | 07-01-2010 |
| | | KR 20090085710 A | 07-08-2009 |
| | | MX PA04009125 A | 07-12-2004 |
| | | RU 2323223 C2 | 27-04-2008 |
| | | ZA 200408511 A | 04-11-2005 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **Bouma BN ; Meijers JC.** Thrombin-activatable fibrinolysis inhibitor. *Journal of Thrombosis and Haemostasis,* 2003, vol. 1, 1566-1574
- **Qiu C. ; De Ronchi D. ; Fratiglioni L.** The epidemiology of the dementias : an update. *Current Opinion in Psychiatry,* 2007, vol. 20, 380-385